# EUROPEAN PATENT APPLICATION

(11) **EP 4 059 498 A1**
(43) Date of publication of application: **21.09.2022**
(21) Application number: 21162892.0
(22) Date of filing: 16.03.2021
(51) Int. Cl.: A61K 31/4184, A61K 31/4439, A61K 31/498, A61K 31/517, A61K 31/519, A61K 31/52, A61P 9/10, A61P 19/00, A61P 19/02, A61P 19/08

(54) **METHODS AND COMPOSITIONS FOR TREATING CONDITIONS ASSOCIATED WITH HYPERMINERALIZATION**

(71) Applicant: Centre Hospitalier Universitaire Vaudois (CHUV), 1011 Lausanne (CH); ZHAW Zurich University for Applied Sciences, 8820 Wädenswil (CH)
(72) Inventor: SO, Alexander, 1009 Pully (CH); BUSSO, Natalie, 1018 Lausanne (CH); NASI, Sonia, 1008 Prilly (CH); BRAND, Michael, 5034 Suhr (CH); GALL, Flavio Max, 8880 Walenstadt (CH); RIEDL, Rainer, 8832 Wilen b. Wollerau (CH)
(74) Representative: KATZAROV S.A.

(57) **Abstract**

The present disclosure relates to novel compounds that are activators of the cystathionine-gamma-lyase enzyme (CSE). The disclosure also relates to methods for preparing the compounds, methods of treatment and/or prevention and to pharmaceutical compositions comprising such compounds.

## Description

### FIELD OF THE INVENTION

The present disclosure relates to novel compounds that are activators of the cystathionine-gamma-lyase enzyme (CSE). The disclosure also relates to methods for preparing the compounds, methods of treatment and/or prevention and to pharmaceutical compositions comprising such compounds.

### BACKGROUND OF THE INVENTION

Heterotopic calcification (HC) is a pathologic process whereby calcium-containing crystals deposit in soft tissues that normally do not calcify such as cartilage, tendons, muscles, skin and others. It often precedes the formation of extra osseous bone, also known as heterotopic ossification (HO) and it exists in acquired and genetic forms, the latter being very rare (Meyers et al. 2019). Acquired HC affects up to 70% of patients who suffer musculoskeletal injuries (trauma or surgery to meniscus, tendons, or the whole joint), central nervous system injuries (spine and brain trauma, strokes), tumors, or severe burns (Ranganathan et al. 2015; Meyers et al. 2019). In addition to pain and limitation of movement, HC can cause additional complications due to increased compression and inflammation of adjacent tissues. In cartilage, HC is considered an etiologic factor in the induction of tissue degradation and plays a pathogenic role in osteoarthritis (OA) (Foreman et al. 2020; Nasi, So, et al. 2016; Stack and McCarthy 2020; Yan et al. 2020). HC is found in 100% of cartilage samples from OA patients undergoing joint replacement surgery (Fuerst et al. 2009). In tendons (rotator cuff and Achilles above all), HC is strongly associated with increased rate of tendon rupture and disability (Oliva, Via, and Maffulli 2012; O'Brien et al. 2012; Michel et al. 2020).

Two families of calcium-containing crystals have been characterized in human cartilage and tendon HC: basic calcium phosphate (BCP) crystals, of which the most common component is hydroxyapatite (HA), and calcium pyrophosphate dihydrate (CPPD) crystals (McCarthy and Dunne 2018). BCP and CPPD are considered damage-associated molecular patterns (DAMPs) as they can trigger deleterious effects in different cell types within joints such as fibroblasts, synoviocytes and macrophages, while less is known about their effect on chondrocytes and tenocytes (McCarthy and Dunne 2018). *In vitro,* BCP and CPPD crystals induced inflammatory responses in musculoskeletal cells, in particular increased secretion of the pro-inflammatory cytokines IL-1β, IL-6, TNF-α and of prostaglandin E₂ (PGE₂) (Liu, Jackson, and Cosgrove 2009; Liu-Bryan and Liote 2005; Nasi, So, et al. 2016; Campillo-Gimenez et al. 2018). BCP and CPPD were shown to induce catabolic responses both in *in vivo* (Easley et al. 2013) and in *in vitro* models, likely via induction of matrix metalloproteinases (*Mmp-1, -3*, *-9* and *-13*) and suppression of tissue inhibitors of Mmp (*Timp-1 and-3*) (Bai et al. 2001; McCarthy et al. 2001; Ryu et al. 2011). In addition to inflammation, calcium-containing crystals are also able to induce cytotoxicity; in particular, CPPD crystals trigger necroptosis (Desai et al. 2017; Mulay et al. 2016) while BCP crystals are more prone to induce apoptosis (Ea et al. 2008). Finally, calcium-containing crystals can trigger oxidative responses via generation of mitochondrial and cytoplasmic reactive oxygen species (ROS) (Campillo-Gimenez et al. 2016; Nasi, Ea, et al. 2016). Those crystal-induced deleterious effects can in turn amplify calcification in musculoskeletal cells, as inflammatory cytokines (Nasi et al. 2015), cell death (Hashimoto et al. 1998) and ROS (Morita et al. 2007; Nasi et al. 2020) have all been demonstrated to be pro-calcifying stimuli. Thus, targeting crystal formation and deposition in musculoskeletal tissues could be of therapeutic relevance in the context of HC.

The treatment of HC has been little studied. Nonsteroidal anti-inflammatory drugs (NSAIDs) or bisphosphonates reduce pain and severity but have no effect on HC. Radiation therapy is an option that has been suggested to interfere with the process of HC formation, but it has late-term side-effects. Currently, the only approach by which already formed HC can be removed is surgery, but complications frequently occur and radiological recurrence rate is estimated around 80% (Meyers et al. 2019).

Hydrogen sulfide (H₂S) is the most recently discovered gasotransmitter after nitric oxide (NO) and carbon monoxide (CO) (Polhemus and Lefer 2014). In mammalian tissues, three different enzymes generate H₂S intracellularly: cystathionine beta-synthase (*CBS*)*,* cystathionine gamma-lyase (*CSE*) and 3-mercaptopyruvate sulfurtransferase (*3-MST*). The enzymes use cysteine as substrate to produce H₂S (Hughes, Centelles, and Moore 2009; Olson 2018), and each has a unique tissue-specific pattern of expression. H₂S has pleiotropic biological effects (Rose, Moore, and Zhu 2017) relevant to HC (Burguera, Meijide-Failde, and Blanco 2017), as in chondrocytes it induces anti-inflammatory and cytoprotective effects (Fox et al. 2012; Kloesch et al. 2012; Nasi et al. 2020) and improves anabolic/catabolic balance (Burguera et al. 2014; Vela-Anero et al. 2017). In addition, we have recently demonstrated that *3-MST*-generated H₂S is important to prevent chondrocyte calcification *in vitro* and HC in an experimental murine model of OA (Nasi et al. 2020). In the same *in vitro* and *in vivo* models, we have demonstrated that thiosulfate (S₂O₃²⁻), an H₂S donor, also had protective effects (Nasi, Ea, et al. 2016). However, direct evidence for beneficial effects of other H₂S-producing enzymes (*CSE* and *CBS*) in HC models are lacking. Suppression of *CSE* almost doubled calcium deposition in the extracellular matrix of human aortic smooth muscle cells *in vitro* (Zavaczki et al. 2011). Concomitant silencing of *CSE* and *CBS* increased human aortic valve calcification (Sikura et al. 2020).

Thus, there is a need for novel therapeutic approaches for the treatment and/or prevention of conditions associated with hypermineralization by augmenting CSE activity via newly developed small molecule CSE specific activators.

### SUMMARY OF THE INVENTION

The present invention relates to a compound of formula (I) 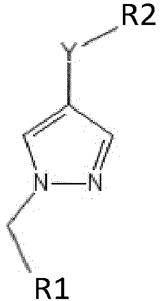 for use in the treatment and/or prevention of a condition associated with hypermineralization, wherein:
R1 is independently alkyl, aryl, cycloalkyl, heteroaryl, or heterocyclyl, optionally substituted;
R2 is nothing or independently ycloalkyl, heteroaryl, or heterocyclyl, optionally substituted; and
Y is alkyl, aryl, cycloalkyl, heteroaryl, or heterocyclyl, preferably, NH, NH2, NO2, CH2 or 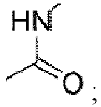
or a pharmaceutically acceptable salt, stereoisomer, mixture of stereoisomers, solvate, or tautomer thereof.

Also provided is a pharmaceutical composition comprising a compound of the invention, or a pharmaceutically acceptable salt thereof, together with a pharmaceutically acceptable carrier for use in the treatment and/or prevention of a condition associated with hypermineralization.

Further provided is a compound of formula (I) 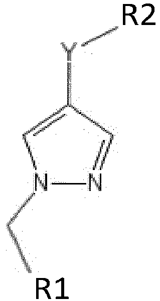 wherein:
R1 is independently alkyl, aryl, cycloalkyl, heteroaryl, or heterocyclyl, optionally substituted;
R2 is nothing or independently cycloalkyl, heteroaryl, or heterocyclyl, optionally substituted; and
Y is alkyl, aryl, cycloalkyl, heteroaryl, or heterocyclyl, preferably NH, NH2, NO2, CH2 or 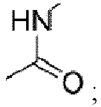
or a salt, stereoisomer, mixture of stereoisomers, solvate, or tautomer thereof.

### DESCRIPTION OF THE FIGURES

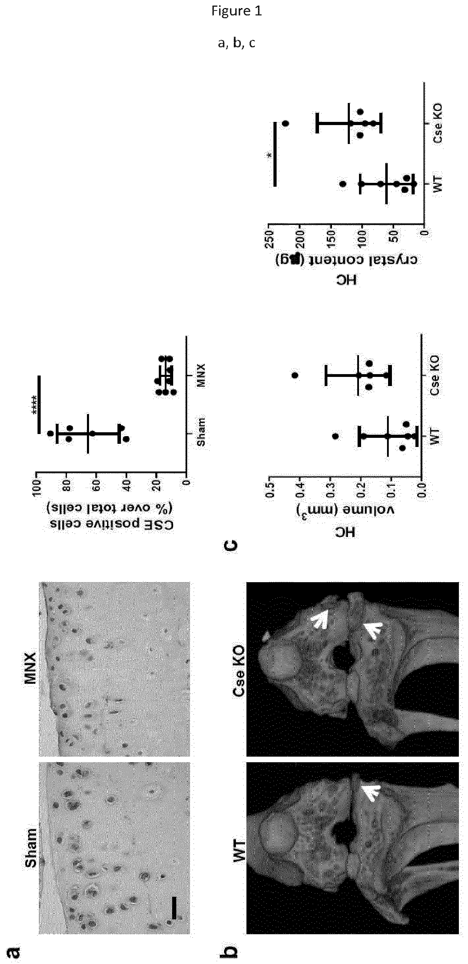

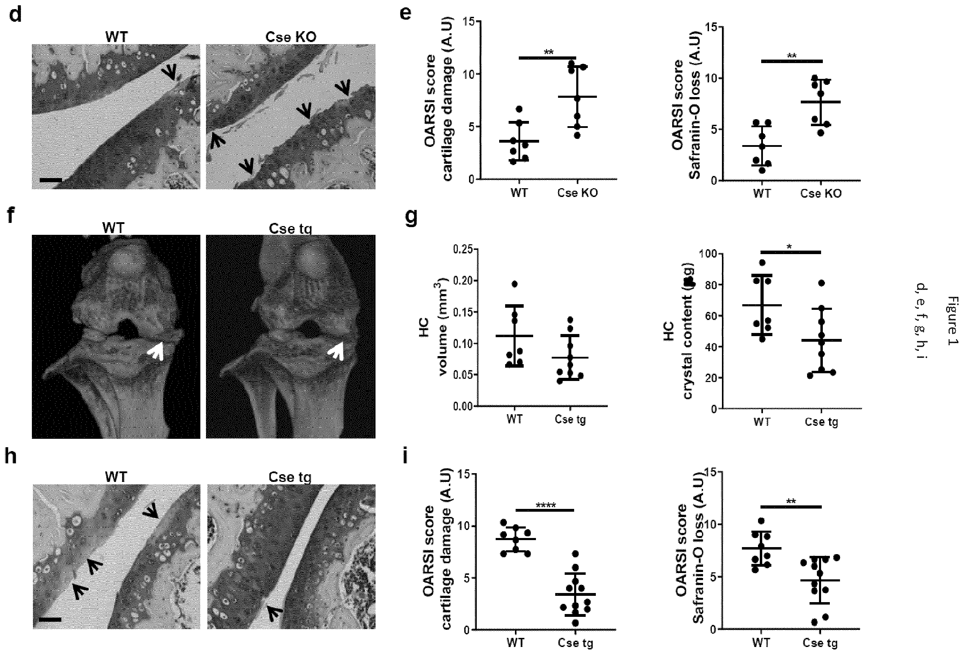

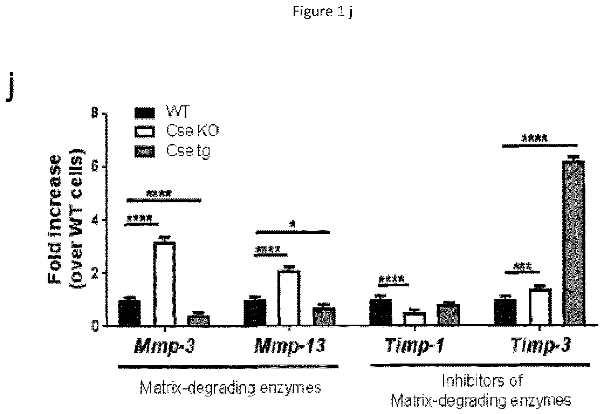

**Figure 1****:** *Cse* deficiency exacerbates joint HC and cartilage damage in experimental OA while *Cse* overexpression is protective
   (**a**) Representative immunohistochemical analysis *of Cse* expression in knee section from sham-operated and MNX WT mice. A knee section from a sham-operated *Cse* KO mouse was used to prove the specificity of the *Cse* antibody. Scale bars 150µm. **** *p*<0.0001. (**b**) Representative micro-CT scan images of WT and *Cse* KO murine knee joints two months after menisectomy (MNX). White arrows show heterotopic calcification in MNX WT knees and its exacerbation in *Cse* KO mice. (**c**) Graphs show CTAnalyzer quantitative analysis of HC volume (mm³) and HC crystal content (µg) in WT and *Cse* KO MNX knees. (d) Representative histologies of WT and *Cse* KO MNX knees, stained with Safranin-O. Black arrows show increased degenerative OA changes in the articular cartilage of *Cse* KO mice. Scale bars 150 µm. (**e**) Graphs show femoral scoring of cartilage damage and Safranin-O loss, accordingly to OARSI method. Mice number WT n=7, *Cse* KO n=7. (**f**) Representative micro-CT scan images of WT and *Cse* tg MNX murine knee joints two months after surgery. White lines show heterotopic calcification in MNX WT knees and its decrease in *Cse* tg mice. (**g**) Graphs show CTAnalyzer quantitative analysis of HC volume (mm³) and HC crystal content (µg) in WT and *Cse* tg MNX knees. (**h**) Representative histologies of WT and *Cse* tg MNX knees, stained with Safranin-O. Black arrows show decreased degenerative OA changes in the articular cartilage of *Cse* tg mice. Scale bars 150µm. (**i**) Graphs show femoral scoring of cartilage damage and Safranin-O loss, accordingly to OARSI method. Mice number WT n=7-8, *Cse* tg n=9-1 1. (**j**qRT-PCR analysis of the basal expression of the indicated genes in WT, *Cse* KO and *Cse* tg chondrocytes. Values obtained in WT cells are considered as reference (=1). **p<0.05,* ** *p*<0.01, **** *p*<0.0001.

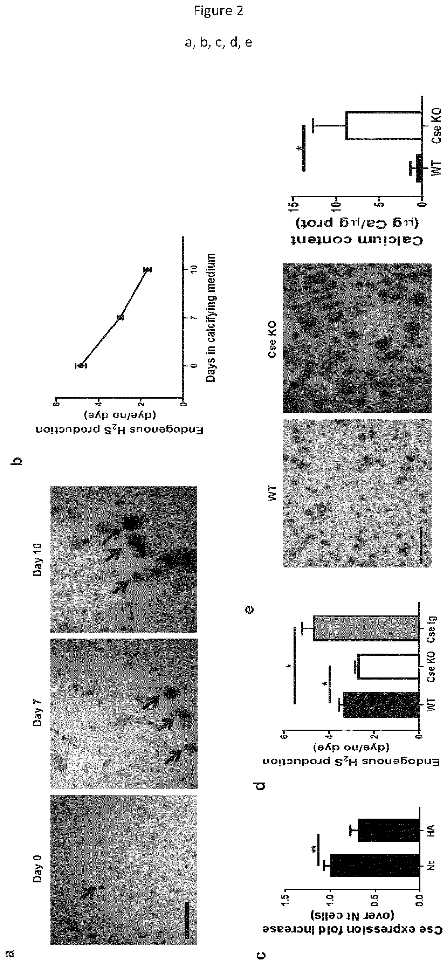

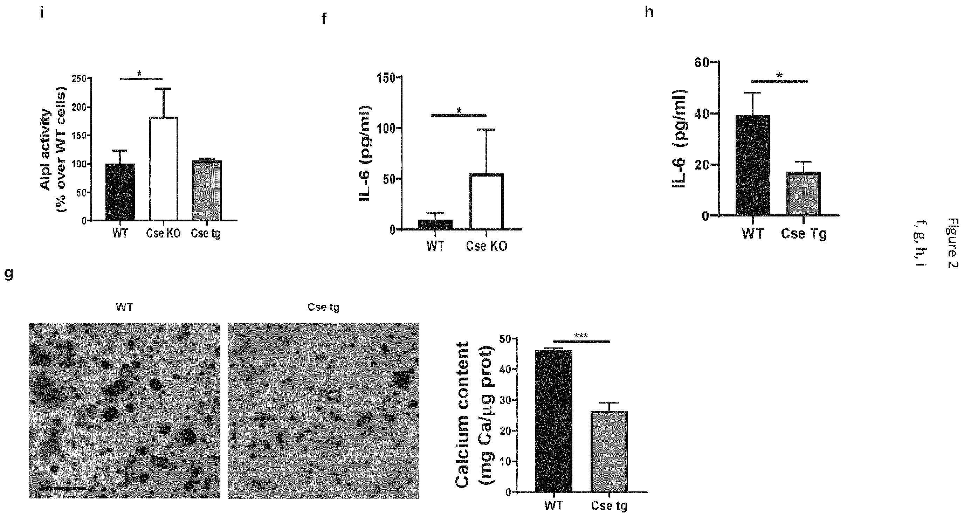

**Figure 2****:** Endogenously produced H₂S regulates chondrocytes calcification and IL-6 secretion
   (**a**) Pictures of chondrocytes cultured in calcifying medium for 0, 7 and 10 days and (**b**) respective FACS analysis of endogenous H₂S production by the P3 probe. (**c**) qRT-PCR for *Cse* gene expression in WT chondrocytes stimulated or not with 500µg/ml HA crystals for 4 hours. (**d**) FACS analysis of endogenous H₂S production by WT, *Cse* KO and *Cse* tg chondrocytes by the P3 probe. (**e**) Alizarin red staining of WT and *Cse* KO chondrocytes cultured in calcification medium for 14 days. Pictures represent triplicates from one experiment of three independent experiments. Graph represents calcium content in the cell monolayer, expressed in mg Ca/µg protein. (**f**) IL-6 secretion by WT and *Cse* KO chondrocytes cultured for 14 days in calcification medium, (**g**) Alizarin red staining of WT and *Cse* tg chondrocytes cultured in calcification medium for 14 days. Pictures represent triplicates from one experiment of three independent experiments. Graph represents calcium content in the cell monolayer, expressed in mg Ca/µg protein. (**h**) IL-6 secretion by WT and *Cse* tg chondrocytes cultured for 14 days in calcification medium, (**i**) Basal Alpl activity in WT, *Cse* Ko and *Cse* tg chondrocytes, expressed as % over WT cells.

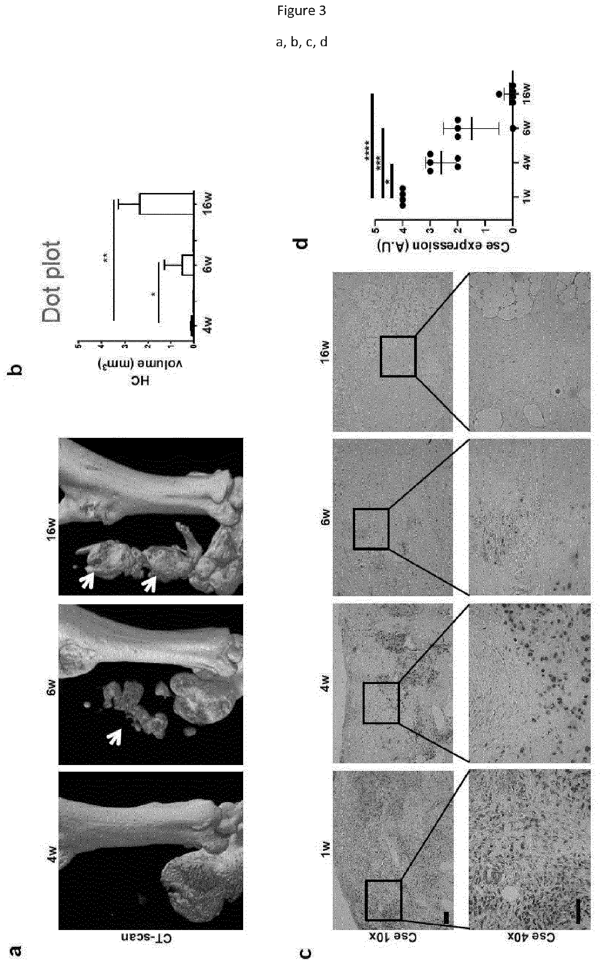

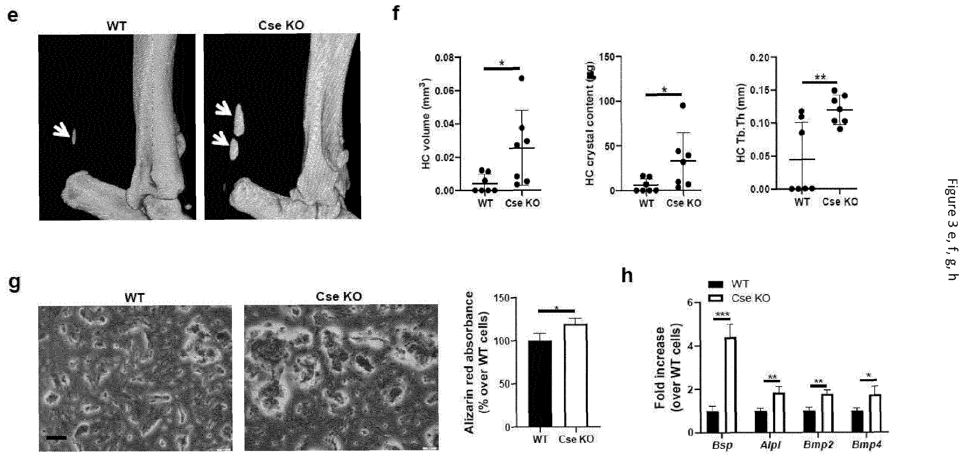

**Figure 3****:** *Cse* expression negatively correlates with surgically induced and spontaneous Achilles tendon HC
   (**a**) Representative micro-CT scan images of WT murine Achilles tendons at 4, 6 and 16 weeks after tenotomy. White arrows show increased HC over time. Np=not performed. (**b**) CTAnalyzer quantitative analysis of HC volume (mm³) over time. n=5 mice/time-point, 15 mice total. (**c**) *Cse* immunohistochemical staining of WT murine Achilles tendons at 1, 4, 6 and 16 weeks after tenotomy. For each time-point, one representative picture from one out of 5 mice is shown. Scale bars 50µm. (**d**) The graph shows the quantification *of Cse* expression in arbitrary units. Mice number n=5 time-point, 20 in total. (**e**) Representative micro-CT scan images of WT and *Cse* KO murine Achilles tendons at 35 weeks of age. White arrows show increased HC in *Cse* KO mice. (**f**) CTAnalyzer quantitative analysis of HC volume (mm³), HC crystal content (µg), and HC trabecular thickness Tb.Th (mm) in WT and *Cse* KO mice. Mice number WT n=7, *Cse* KO n=7. (**g**) Alizarin red staining of WT and *Cse* KO tenocytes cultured in calcification medium for 24hours. Pictures represent triplicates from one experiment of three independent experiments. Graph represents Alizarin red quantification, expressed in % over WT cells. (**h**) qRT-PCR analysis of the basal expression of the indicated genes in WT and *Cse* KO tenocytes. Values obtained in WT cells are considered as reference (=1) **p<0.05,* ** p<0. 0 1, *** p<0.001, **** p<0.0001.

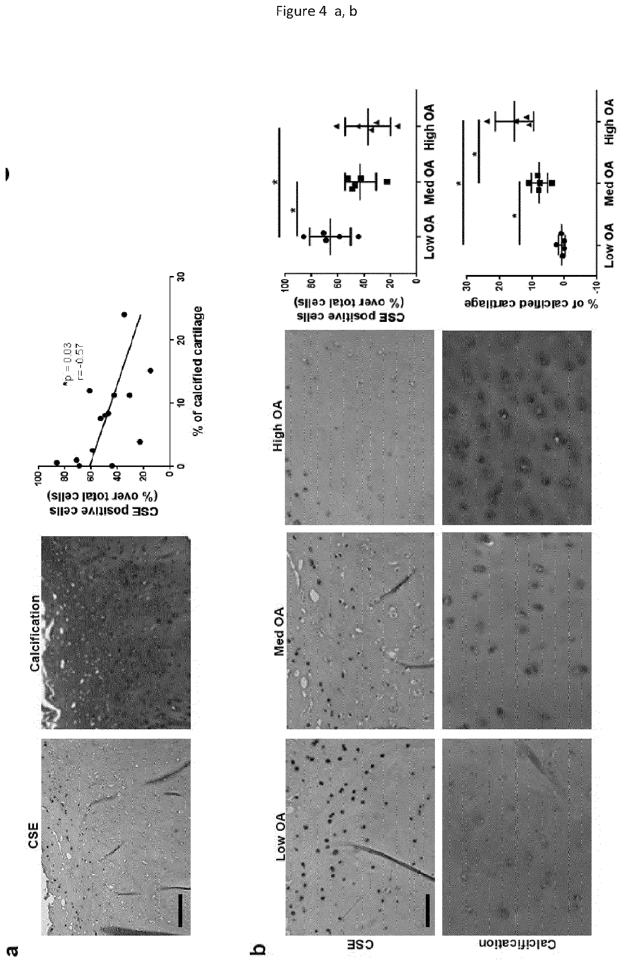

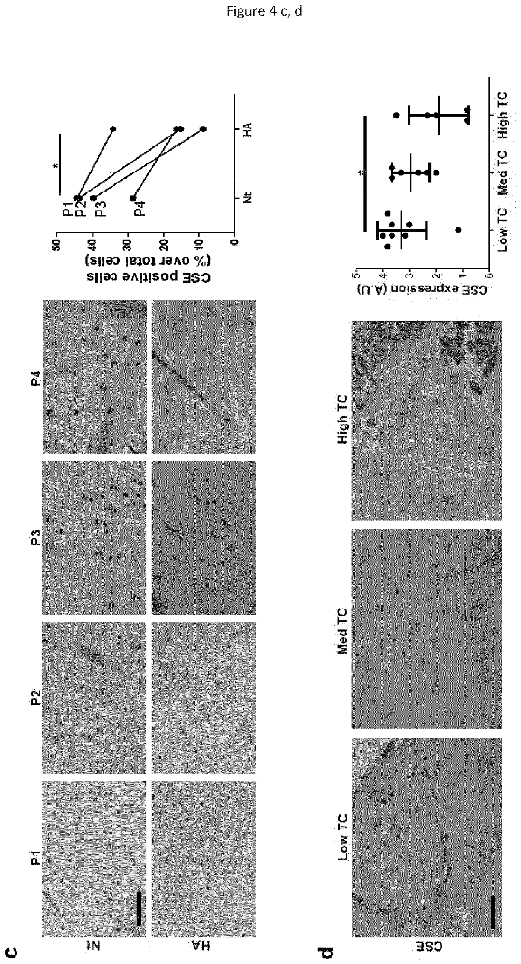

**Figure 4****:** Cartilage *CSE* expression negatively correlates with chondrocyte calcification and OA severity, and is inhibited by HA crystals
   (**a**) *CSE* immunohistochemical staining in cartilage from individuals with osteoarthritis and consecutive sections stained with von Kossa/Safranin-O staining for phosphate containing crystals. For each staining, one representative picture from one out of 14 patients is shown. Scale bars 200µm. The graph shows the correlation between the % of *CSE* positive cells and the % of calcified cartilage in the different patients. n=14 patients. (**b**) *CSE* immunohistochemical staining in cartilage from individuals with low, medium and high stage osteoarthritis and consecutive sections stained with von Kossa/Safranin-O staining for phosphate containing crystals. For each staining, one representative picture from one out of 5 patients per group is shown. Scale bars 200µm. Graphs show quantification of the % of *CSE* positive cells and the % of calcified cartilage. For each patient, three fields were counted per explant and the mean plotted in the graph. n=15 patients. (**c**) *CSE* immunohistochemical staining in human cartilage explants stimulated with 500µg/ml HA crystals or let untreated (Nt) for 24 hours. Scale bars 200µm. The graph shows the % *of CSE* positive cells in Nt and HA-stimulated explants in the different patients. For each patient, three fields were counted per explant and the mean plotted in the graph. Lines connect the Nt condition to the HA condition for each patient. n=4 patients (P1-P4). **p<*0.05. (**d**) *CSE* immunohistochemical staining in tendons from individuals with low, medium and high calcific tendinopathy. One representative picture from one out of 5 to 8 patients per group is shown. Scale bars 200µm. Graphs show quantification *of CSE* expression. For each patient, three fields were counted per explant and the mean plotted in the graph. n=19 patients.

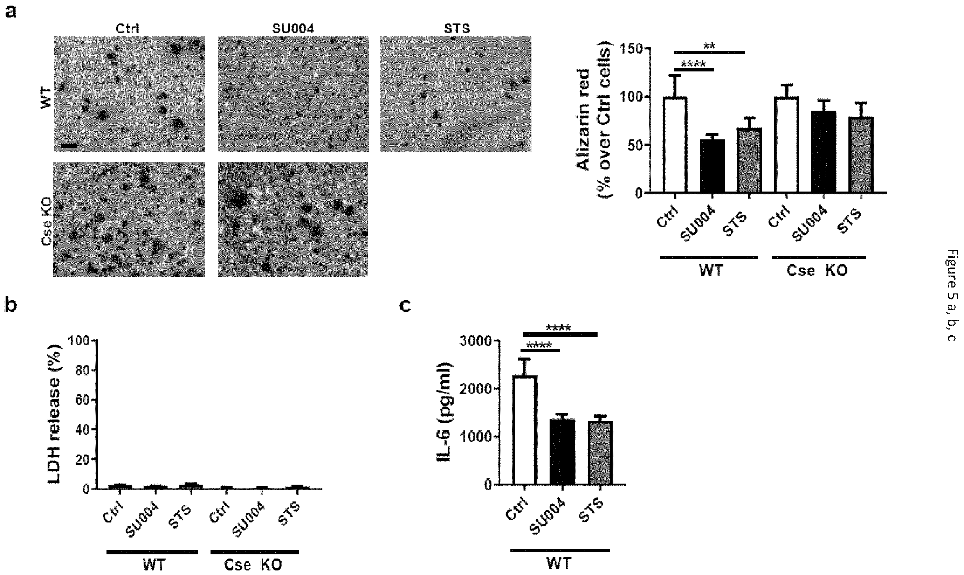

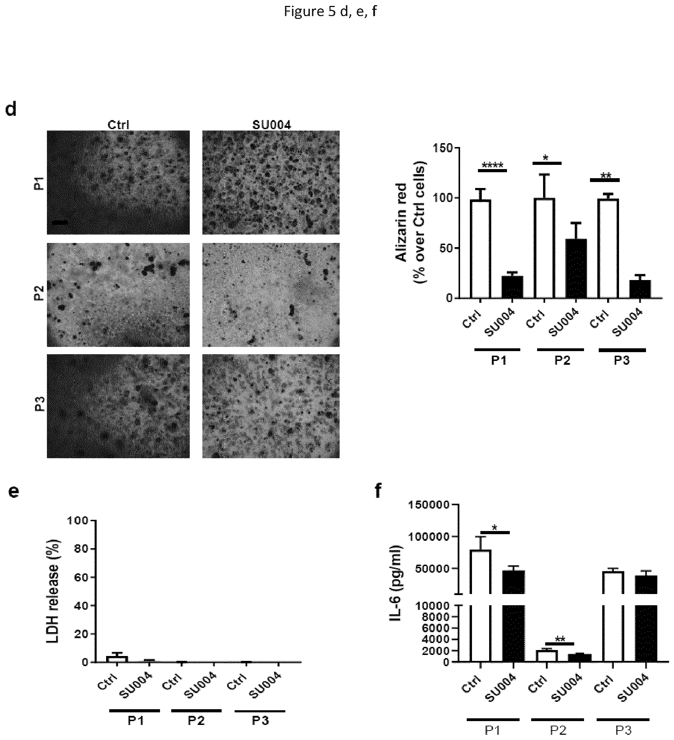

**Figure 5****:** A new small molecule CSE activator compound inhibits chondrocyte mineralization and IL-6 production.
   (**a**) WT and Cse-deficient chondrocytes were incubated with a *CSE* activator (SU004, 50µM) previously identified by high-through put screen, or with the equivalent volume of DMSO (0,1%) for 24 hours in calcification medium. STS 25mM was used as a positive control for calcification inhibition. Calcium-containing crystals were stained with Alizarin red and quantified. (**b**) Cytotoxicity was assessed by LDH measurement and expressed as % over 100% cytotoxicity. (**c**) IL-6 secretion was measured by ELISA in supernatant of WT chondrocytes incubated with SU004 50µM or STS 25mM for 24 hours in calcification medium, (**d**) Primary human chondrocytes from 3 OA patients (P1, P2, P3) were incubated with SU004 50µM, or with the equivalent volume of DMSO (0,1%) for 24 hours in calcification medium. Calcium-containing crystals were stained with Alizarin red and quantified. (**e**) Cytotoxicity was assessed by LDH measurement and expressed as % over 100% cytotoxicity. (**f**) IL-6 secretion was measured by ELISA in supernatant of human chondrocytes incubated for 24 hours with SU004 50µM in calcification medium. **p<0.05,* ** *p*<0.01, *****p*<0.0001.

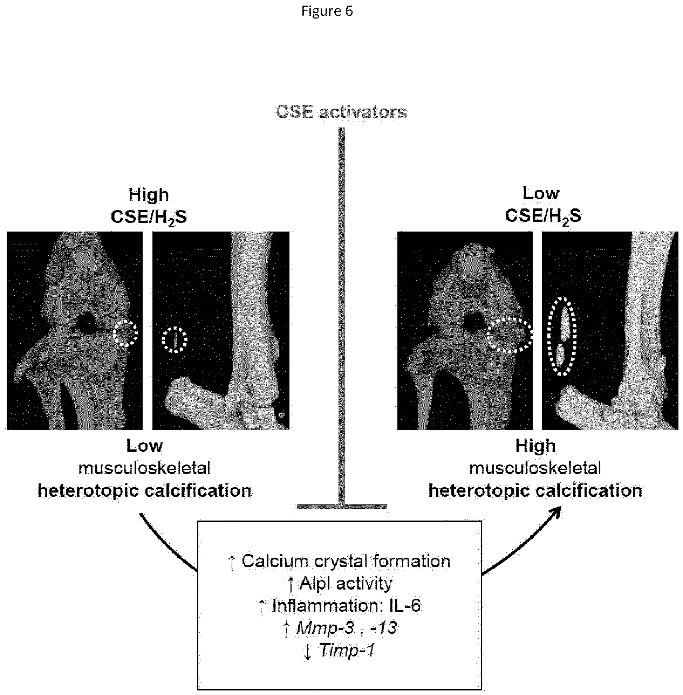

**Figure 6****:** Proposed mechanism for *CSE* involvement in heterotopic ossification in musculoskeletal tissues
   High *CSE* expression, and therefore high levels of H₂S, in knee cartilage and Achilles tendons is associated with low heterotopic ossification in these musculoskeletal tissues. On the other hand, low *CSE* expression and decreased H₂S levels, favors increased heterotopic ossification in musculoskeletal tissues. In particular, we described that lack *of CSE* sustains chondrocyte mineralization *in vitro* and joint calcification *in vivo,* partly via increased Alp activity. In addition, low *CSE* expression is associated with increased IL-6, that participates in amplifying chondrocytes calcification as previously described (Nasi, So, et al. 2016). Finally, low *CSE* combines with increased *Mmp-3* and *Mmp-13* expression by chondrocytes *in vitro,* leading to cartilage degradation. Restoring physiological levels of H₂S in joints by *CSE* activators can block chondrocyte calcification and IL-6 production and could therefore be of therapeutic relevance for the treatment of heterotopic ossification in musculoskeletal tissues.

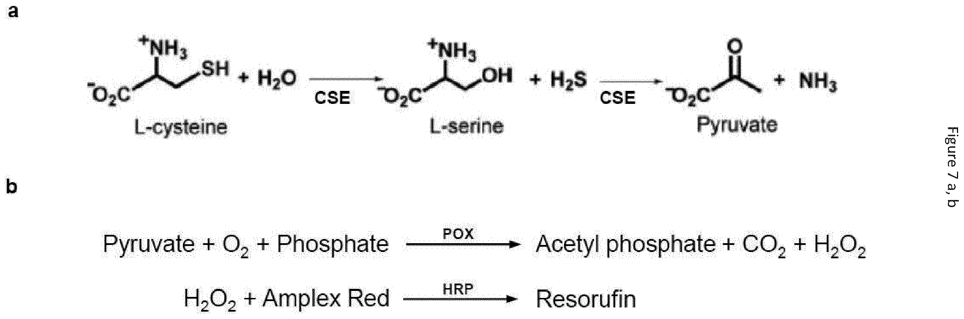

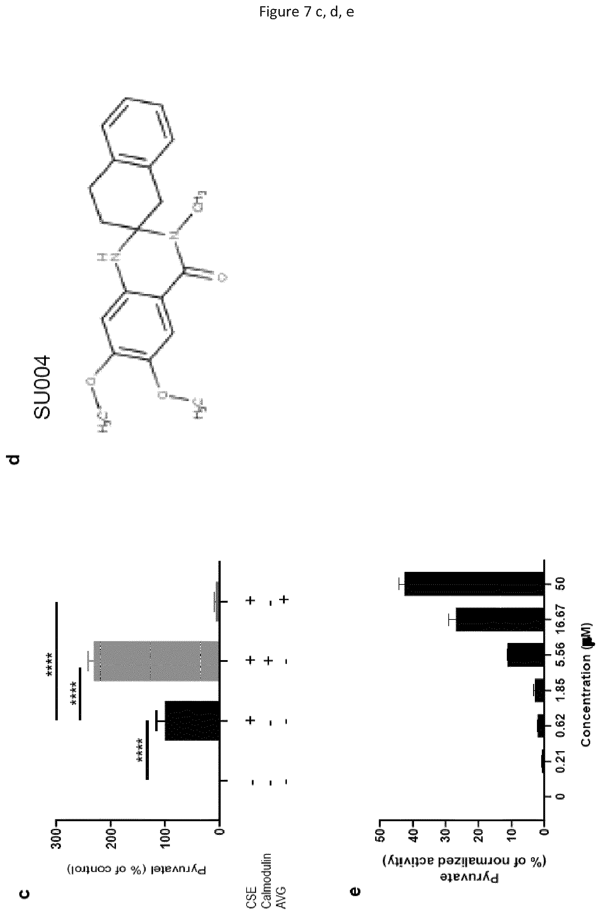

**Figure 7****:** Set-up of the high-throughput screening assay and identification of a *CSE* activator compound.
   The chemical reactions involved in the 2 step assay are depicted. In (**a**) recombinant human *CSE* (*hCSE*) is incubated with L-cysteine, generating H₂S and pyruvate. In (**b**) pyruvate is oxidized by pyruvate oxidase (POX) to generate acetate, carbon dioxide (CO₂) and hydrogen peroxide (H₂O₂). H₂O₂ then reacts with Amplex Red in a 1:1 stoichiometry to form the red fluorescent product, resorufin. (**c**) *hCSE*-dependent pyruvate measurement without enzyme (no pyruvate production), with the specific *CSE* inhibitor AVG 5 µM (inhibition of pyruvate production) and with the *CSE* activator calmodulin 5 µM (increased pyruvate production). Results were expressed as the % of pyruvate generated over *hCSE* alone. (**d**) Chemical structure of the new small molecule *CSE* activator SU004. (**e**) *CSE*-dependent pyruvate upon incubation *of hCSE* with L-cysteine and with increasing concentration of SU004. The ability of SU004 to activate *hCSE* was calculated as % of normalized activity.

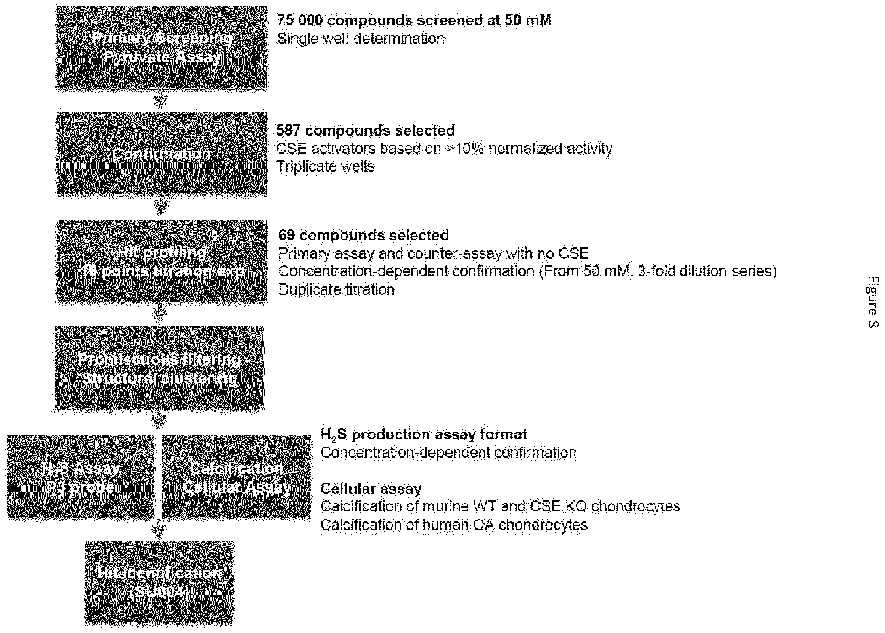

**Figure 8****:** Flow-chart for the high-throughput screening of CSE modulators.
**Figure 9****:** C5 and Z20 augments CSE production of H2S
   The activity of recombinant human CSE (rCSE) was tested in the presence of C5 (50uM), Z20 (50uM) or vehicle (DMSO). Briefly, compounds were added in reaction buffer (50 mM Tris-HCl, pH 8) containing 2µg of rCSE, 10mM CSE substrate L-cysteine, 5uM pyridoxal 5'-phosphate (PLP). After addition of 10 µM of the H2S sensitive fluorescent probe 7-azido-4-methylcoumarin (AzMC) in a total volume of 100 µl, fluorescence was immediately measured in kinetic mode for 2 h at 37 °C with SpectraMax M5 reader, with excitation and emission wavelengths of 365 nm and 450 nm, respectively. Statistical analysis was determined using simple linear regression with p<0.0001 for C5 vs. vehicle and p<0.0002 for Z20 vs. vehicle.

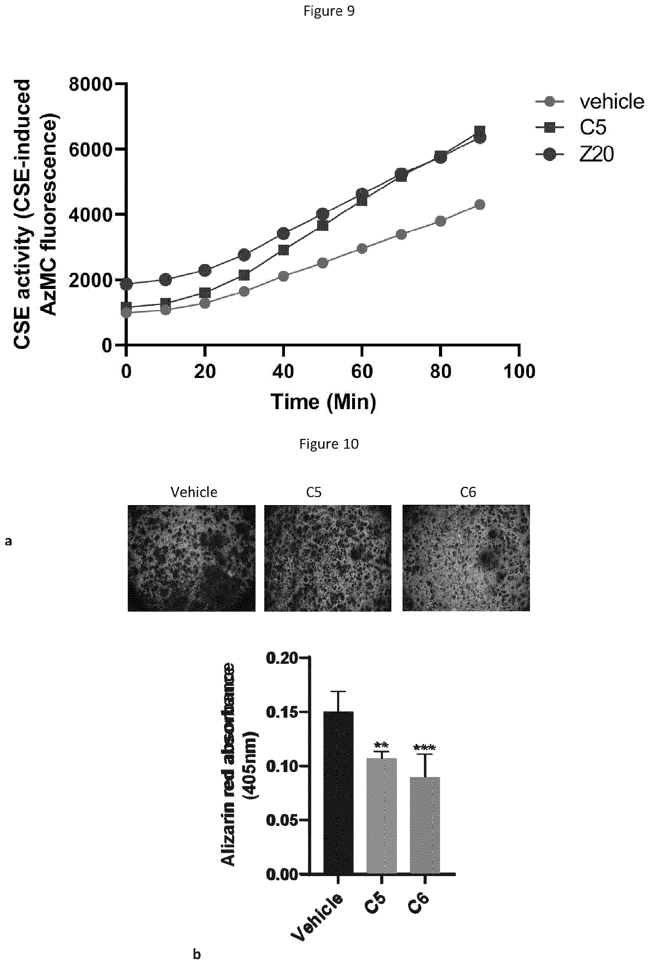

**Figure 10****:** C5 and C6 inhibit primary murine chondrocyte mineralization in vitro
   Primary murine chondrocytes were stimulated with secondary calciprotein particles (sCPP) for 24 h in DMEM + 10% FBS in presence of C5 or C6 at 50uM or vehicle (DMSO). Mineralization was analyzed by Alizarin red staining of the cells (A) and quantified (B). Statistical analysis was determined using one-way ANOVA with **p < 0.01 and ***p < 0.001.

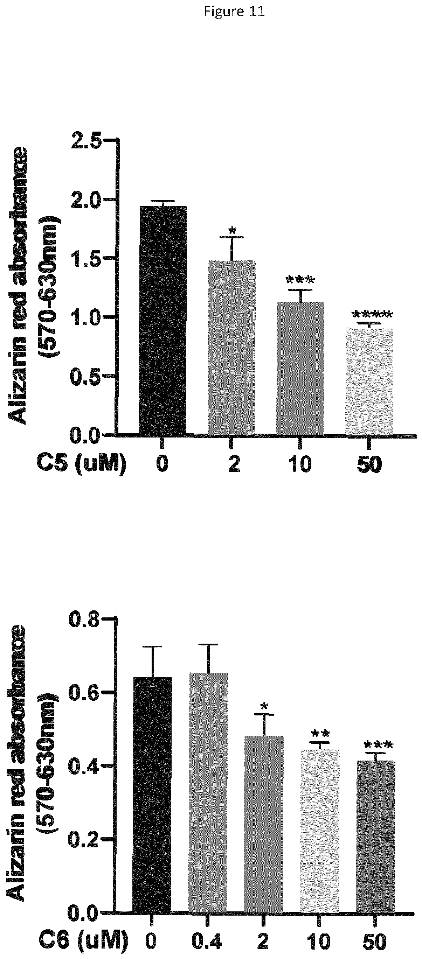

**Figure 11****:** C5 and C6 inhibit ATDC5 mineralization in vitro in a dose dependent fashion
   Alizarin red staining of ATDC5 chondrocytes stimulated with sCPP for 24 h in DMEM + 10% FBS in presence of different concentrations of compounds C5 and C6. The graphs show Alizarin red absorbance at 570-630nm. Statistical analysis (in comparison to 0) was determined using one-way ANOVA with *p < 0.05, **p < 0.01, ***p < 0.001, ****p < 0.001.

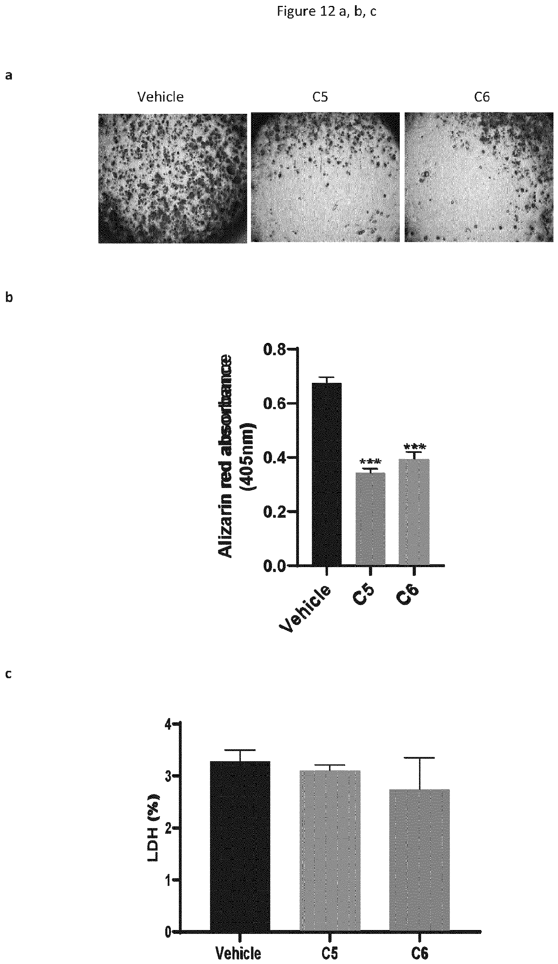

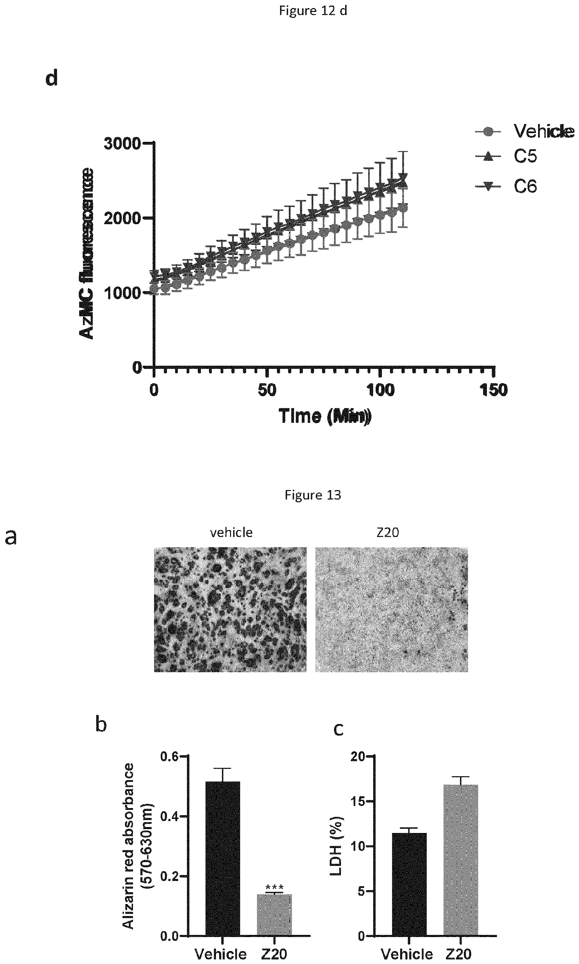

**Figure 12****:** C5 and C6 inhibit murine vascular smooth muscle cells (MOVAS cells) mineralization
   A) Alizarin red staining of MOVAS cells stimulated with sCPP for 24 h in DMEM + 10% FBS and treated or not with 50 uM of compounds. B) The graph shows Alizarin red absorbance at 405nm. Statistical analysis was determined using one-way ANOVA with ***p < 0.001. C) Percentage of cytotoxicity (LDH %) in MOVAS treated or not with compounds for 24h. D) MOVAS cells were seeded onto half-area 96-well plate and incubated overnight. Then C5 or C6 at 50uM or vehicle (DMSO), L-cysteine at 10mM, PLP at 5uM and the H2S sensitive fluorescent probe AzMC at 10 µM were added. The fluorescence was immediately measured in kinetic mode for 2 h at 37 °C with SpectraMax M5 reader, with excitation and emission wavelengths of 365 nm and 450 nm, respectively.
**Figure 13****::** Z20 inhibits MOVAS cells mineralization in vitro
   A) Alizarin red staining of Movas cells stimulated with sCPP for 24 h in DMEM + 10% FBS and treated or not with 50 uM of Z20. B) The graph shows Alizarin red absorbance at 570-630. C) Percentage of cytotoxicity (LDH %) in murine chondrocytes treated or not with Z20 for 24h. Statistical analysis was determined using one-way ANOVA with ***p < 0.001.

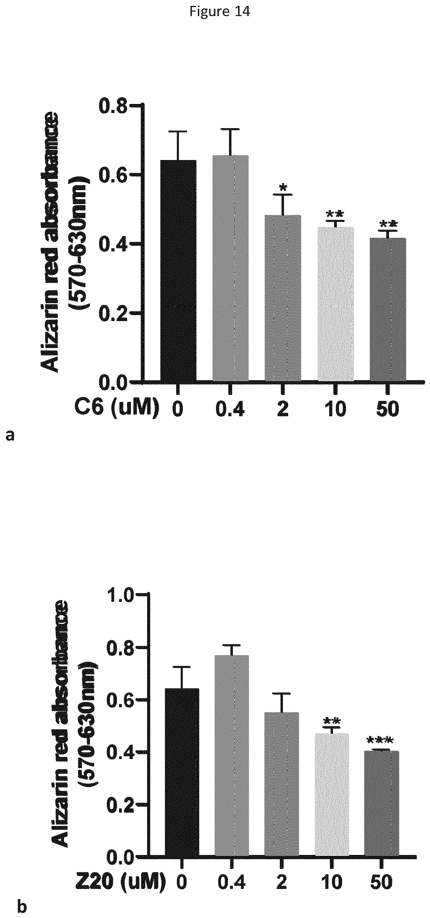

**Figure 14****:** Dose-dependent effect of C6 and Z20 on MOVAS mineralization
   Alizarin red staining of MOVAS cells stimulated with sCPP for 24 h in DMEM + 10% FBS and treated or not with different concentrations of compounds (A: C6) and (B: Z20). The graphs show Alizarin red absorbance at 570-630nm. Statistical analysis was determined using one-way ANOVA with *p < 0.05, **p < 0.01, ***p < 0.001.

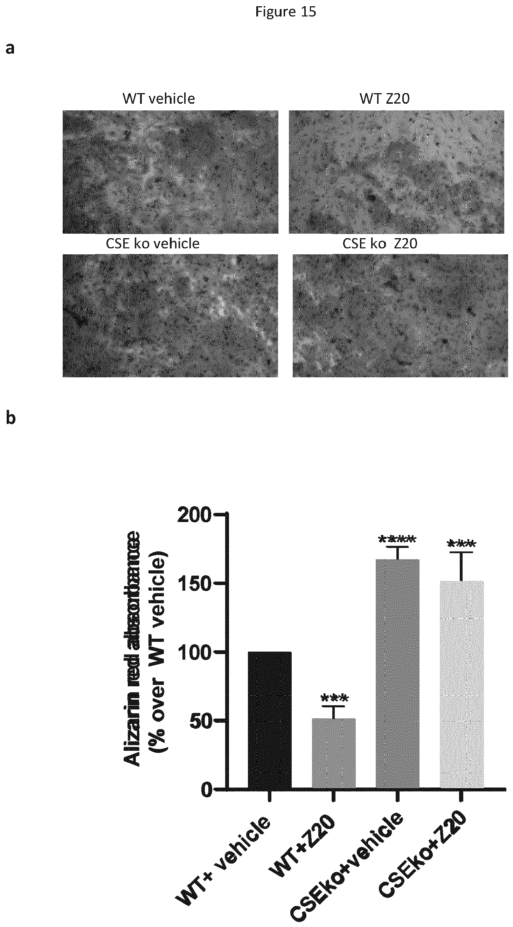

**Figure 15****:** Z20 inhibits primary vascular smooth muscle cell mineralization in vitro
   Primary VSMC were isolated from wild-type (WT) or CSE-deficient (CSE ko) mice. A) Alizarin red staining of WT or CSE ko VSMC stimulated with secondary CPP for 24 h in DMEM + 10% FBS and treated or not with 50 uM of compound Z20. B) The graph shows Alizarin red absorbance at 570-630nm expressed in % over WT vehicle. Statistical analysis was determined using one-way ANOVA (compared to WT vehicle) with ***p < 0.001, ****p < 0.0001.

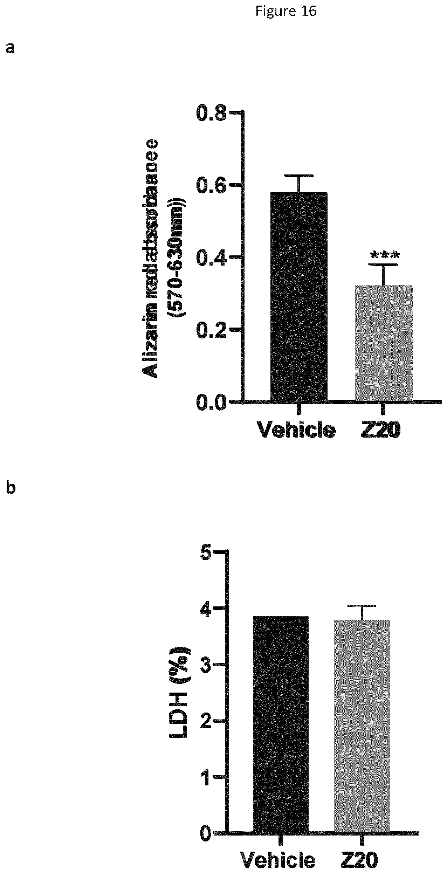

**Figure 16****:** Z20 inhibits primary murine tenocyte mineralization in vitro
   A) Alizarin red absorbance at 570-630nm of primary murine tenocytes stimulated with secondary CPP for 24 h in DMEM + 10% FBS and treated or not with 50 uM of Z20. B) Percentage of cytotoxicity (LDH %) in tenocytes treated or not with Z20 for 24h. Statistical analysis was determined using one-way ANOVA with ***p < 0.001.

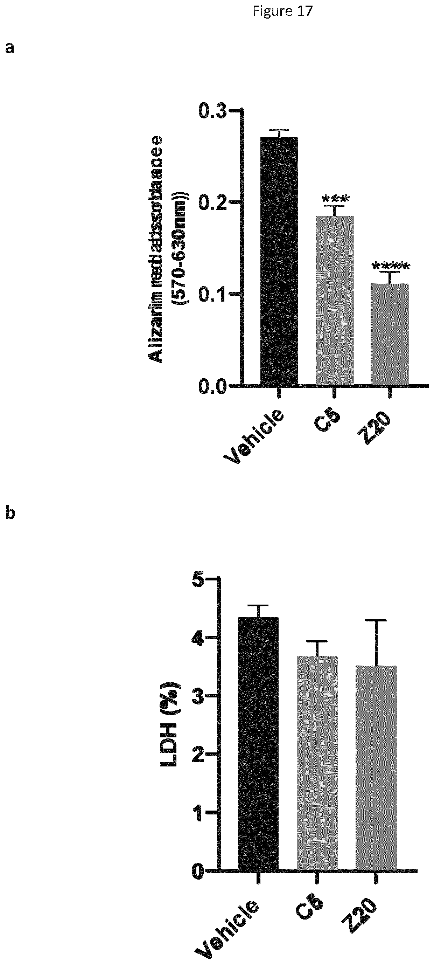

**Figure 17****:** C5 and Z20 inhibit primary human chondrocyte mineralization
   A) Alizarin red staining of primary human chondrocytes stimulated with secondary CPP for 24 h in DMEM + 10% FBS and treated or not with 50 uM of compounds.
   B) B) Percentage of cytotoxicity (LDH %) in human chondrocytes treated or not with compounds for 24h. Statistical analysis was determined using one-way ANOVA with ***p < 0.001, ****p < 0.0001.

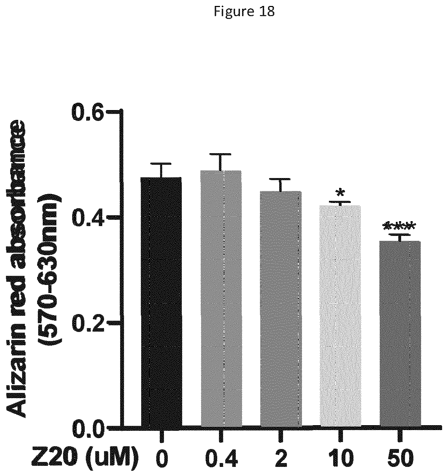

**Figure 18****:** Z20 inhibits human keratinocyte line mineralization
   Immortalized human keratinocytes HaCaT cells were stimulated with secondary CPP and different concentrations of the compound Z20 for 24 h. Cells were then stained with Alizarin Red. The graph shows Alizarin red absorbance at 570-630nm. Statistical analysis was determined using one-way ANOVA with *p < 0.05, ***p < 0.001.

### DESCRIPTION OF THE INVENTION

Although methods and materials similar or equivalent to those described herein can be used in the practice or testing of the present invention, suitable methods and materials are described below. All publications, patent applications, patents, and other references mentioned herein are incorporated by reference in their entirety. The publications and applications discussed herein are provided solely for their disclosure prior to the filing date of the present application. Nothing herein is to be construed as an admission that the present invention is not entitled to antedate such publication by virtue of prior invention. In addition, the materials, methods, and examples are illustrative only and are not intended to be limiting.

In the case of conflict, the present specification, including definitions, will control. Unless defined otherwise, all technical and scientific terms used herein have the same meaning as is commonly understood by one of skill in art to which the subject matter herein belongs. As used herein, the following definitions are supplied in order to facilitate the understanding of the present invention.

Reference throughout this specification to "one aspect", "an aspect", "another aspect", "a particular aspect", "combinations thereof" means that a particular feature, structure or characteristic described in connection with the invention aspect is included in at least one aspect of the present invention. Thus, the appearances of the foregoing phrases in various places throughout this specification are not necessarily all referring to the same aspect. Furthermore, the particular features, structures, or characteristics may be combined in any suitable manner in one or more aspects.

The term "comprise(s)", or "comprising" is generally used in the sense of include(s)/including, that is to say, permitting the presence of one or more features or components. The terms "comprise(s)" and "comprising" also encompass the more restricted ones "consist(s)" and "consisting", respectively.

As used in the specification and claims, the singular form "a", "an" and "the" include plural references unless the context clearly dictates otherwise.

The prefix "Cᵤ₋ᵥ" indicates that the following group has from u to v carbon atoms. For example, "C₁₋₆ alkyl" indicates that the alkyl group has from 1 to 6 carbon atoms.

"Alkyl" refers to an unbranched or branched saturated hydrocarbon chain. As used herein, alkyl has 1 to 20 carbon atoms (i.e., C₁₋₂₀ alkyl), 1 to 8 carbon atoms (i.e., C₁₋₈ alkyl), 1 to 6 carbon atoms (i.e., C₁₋₆ alkyl), or 1 to 4 carbon atoms (i.e., C₁₋₄ alkyl). Examples of alkyl groups include methyl, ethyl, propyl, isopropyl, n-butyl, sec-butyl, iso-butyl, tert-butyl, pentyl, 2-pentyl, isopentyl, neopentyl, hexyl, 2-hexyl, 3-hexyl, and 3-methylpentyl. When an alkyl residue having a specific number of carbons is named by chemical name or identified by molecular formula, all positional isomers having that number of carbons may be encompassed; thus, for example, "butyl" includes n-butyl (i.e., -(CH₂)₃CH₃), sec-butyl (i.e., - CH(CH₃)CH₂CH₃), isobutyl (i.e., -CH₂CH(CH₃)₂) and tert-butyl (i.e., -C(CH₃)₃); and "propyl" includes n-propyl (i.e., -(CH₂)₂CH₃) and isopropyl (i.e., -CH(CH₃)₂).

"Aryl" refers to an aromatic carbocyclic group having a single ring (e.g., monocyclic) or multiple rings (e.g., bicyclic or tricyclic) including fused systems. As used herein, aryl has 6 to 20 ring carbon atoms (i.e., C₆₋₂₀ aryl), 6 to 12 carbon ring atoms (i.e., C₆₋₁₂ aryl), or 6 to 10 carbon ring atoms (i.e., C₆₋₁₀ aryl). Examples of aryl groups include, e.g., phenyl, naphthyl, fluorenyl and anthryl. Aryl, however, does not encompass or overlap in any way with heteroaryl defined below. If one or more aryl groups are fused with a heteroaryl, the resulting ring system is heteroaryl. If one or more aryl groups are fused with a heterocyclyl, the resulting ring system is heterocyclyl.

"Cycloalkyl" refers to a saturated or partially unsaturated cyclic alkyl group having a single ring or multiple rings including fused, bridged and spiro ring systems. The term "cycloalkyl" includes cycloalkenyl groups (i.e., the cyclic group having at least one double bond) and carbocyclic fused ring systems having at least one sp³ carbon atom (i.e., at least one non-aromatic ring). As used herein, cycloalkyl has from 3 to 20 ring carbon atoms (i.e., C₃₋₂₀ cycloalkyl), 3 to 12 ring carbon atoms (i.e., C₃₋₁₂ cycloalkyl), 3 to 10 ring carbon atoms (i.e., C₃₋₁₀ cycloalkyl), 3 to 8 ring carbon atoms (i.e., C₃₋₈ cycloalkyl), or 3 to 6 ring carbon atoms (i.e., C₃₋₆ cycloalkyl). Monocyclic groups include, for example, cyclopropyl, cyclobutyl, cyclopentyl, cyclohexyl, cycloheptyl and cyclooctyl. Further, the term cycloalkyl is intended to encompass any non-aromatic ring which may be fused to an aryl ring, regardless of the attachment to the remainder of the molecule. Still further, cycloalkyl also includes "spirocycloalkyl" when there are two positions for substitution on the same carbon atom.

"Heteroaryl" refers to an aromatic group having a single ring, multiple rings or multiple fused rings, with one or more ring heteroatoms independently selected from nitrogen, oxygen, and sulfur. As used herein, heteroaryl includes 1 to 20 ring carbon atoms (i.e., C₁₋₂₀ heteroaryl), 3 to 12 ring carbon atoms (i.e., C₃₋₁₂ heteroaryl), or 3 to 8 carbon ring atoms (i.e., C₃₋₈ heteroaryl), and 1 to 5 ring heteroatoms, 1 to 4 ring heteroatoms, 1 to 3 ring heteroatoms, 1 to 2 ring heteroatoms, or 1 ring heteroatom independently selected from nitrogen, oxygen and sulfur. In certain instances, heteroaryl includes 5-10 membered ring systems, 5-7 membered ring systems, or 5-6 membered ring systems, each independently having 1 to 4 ring heteroatoms, 1 to 3 ring heteroatoms, 1 to 2 ring heteroatoms, or 1 ring heteroatom independently selected from nitrogen, oxygen and sulfur. Any aromatic ring, having a single or multiple fused rings, containing at least one heteroatom, is considered a heteroaryl regardless of the attachment to the remainder of the molecule (i.e., through any one of the fused rings). Heteroaryl does not encompass or overlap with aryl as defined above.

"Heterocyclyl" refers to a saturated or partially unsaturated cyclic alkyl group, with one or more ring heteroatoms independently selected from nitrogen, oxygen and sulfur. The term "heterocyclyl" includes heterocycloalkenyl groups (i.e., the heterocyclyl group having at least one double bond), bridged-heterocyclyl groups, fused-heterocyclyl groups and spiroheterocyclyl groups. A heterocyclyl may be a single ring or multiple rings wherein the multiple rings may be fused, bridged or spiro, and may comprise one or more (e.g., 1 to 3) oxo (=O) or N-oxide (-O⁻) moieties. Any non-aromatic ring containing at least one heteroatom is considered a heterocyclyl, regardless of the attachment (i.e., can be bound through a carbon atom or a heteroatom). Further, the term heterocyclyl is intended to encompass any non-aromatic ring containing at least one heteroatom, which ring may be fused to an aryl or heteroaryl ring, regardless of the attachment to the remainder of the molecule. As used herein, heterocyclyl has 2 to 20 ring carbon atoms (i.e., C₂₋₂₀ heterocyclyl), 2 to 12 ring carbon atoms (i.e., C₂₋₁₂ heterocyclyl), 2 to 10 ring carbon atoms (i.e., C₂₋₁₀ heterocyclyl), 2 to 8 ring carbon atoms (i.e., C₂₋₈ heterocyclyl), 3 to 12 ring carbon atoms (i.e., C₃₋₁₂ heterocyclyl), 3 to 8 ring carbon atoms (i.e., C₃₋₈ heterocyclyl), or 3 to 6 ring carbon atoms (i.e., C₃₋₆ heterocyclyl); having 1 to 5 ring heteroatoms, 1 to 4 ring heteroatoms, 1 to 3 ring heteroatoms, 1 to 2 ring heteroatoms, or 1 ring heteroatom independently selected from nitrogen, sulfur or oxygen. The term "heterocyclyl" also includes "spiroheterocyclyl" when there are two positions for substitution on the same carbon atom.

The terms "optional" or "optionally" means that the subsequently described event or circumstance may or may not occur, and that the description includes instances where said event or circumstance occurs and instances in which it does not. Also, the term "optionally substituted" refers to any one or more hydrogen atoms on the designated atom or group may or may not be replaced by a moiety other than hydrogen. "Optionally substituted" may be zero to the maximum number of possible substitutions, and each occurrence is independent. When the term "substituted" is used, then that substitution is required to be made at a substitutable hydrogen atom of the indicated substituent. An optional substitution may be the same or different from a (required) substitution.

When a moiety is "optionally substituted," and reference is made to a general term, such as any "alkyl," "alkenyl," "alkynyl," "haloalkyl," "cycloalkyl," "aryl" or "heteroaryl," then the general term can refer to any antecedent specifically recited term, such as (C₁₋₃ alkyl), (C₄₋₆ alkyl), -O(C₁₋₄ alkyl), (C₃₋₁₀ cycloalkyl), O-(C₃₋₁₀ cycloalkyl) and the like. For example, "any aryl" includes both "aryl" and as well as examples of aryl, such as phenyl or naphthyl and the like. Also, the term "any heterocyclyl" includes heterocyclyls, such as oxetanyl, tetrahydropyranyl, morpholino, piperidinyl and the like. In the same manner, the term "any heteroaryl" includes heteroaryls, such as pyridine, pyridazine, thiazole, thiadiazole, quinoline and the like.

Provided also are stereoisomers, mixture of stereoisomers, tautomers, solvates, isotopically enriched analog, and pharmaceutically acceptable salts of the compounds described herein.

The compounds disclosed herein, or their pharmaceutically acceptable salts, may include an asymmetric center and may thus give rise to enantiomers, stereoisomer, diastereomers, and other stereoisomeric forms that may be defined, in terms of absolute stereochemistry, as (*R*)- or (*S*)- or, as (D)- or (L)- for amino acids. The present disclosure is meant to include all such possible isomers, as well as their racemic and optically pure forms. Optically active (+) and (-), (*R*)- and (*S*)-, or (D)- and (L)- isomers may be prepared using chiral synthons or chiral reagents, or resolved using conventional techniques, for example, chromatography and fractional crystallization. Conventional techniques for the preparation/isolation of individual enantiomers include chiral synthesis from a suitable optically pure precursor or resolution of the racemate (or the racemate of a salt or derivative) using, for example, chiral high pressure liquid chromatography (HPLC). When the compounds described herein contain olefinic double bonds or other centers of geometric asymmetry, and unless specified otherwise, it is intended that the compounds include both E and Z geometric isomers.

A "stereoisomer" refers to a compound made up of the same atoms bonded by the same bonds but having different three-dimensional structures, which are not interchangeable. The present disclosure contemplates various stereoisomers and mixtures thereof and includes "enantiomers," which refers to two stereoisomers whose molecules are non-superimposable mirror images of one another and "diastereomers," which refers to stereoisomers that have at least two asymmetric atoms, but which are not mirror-images of each other. Thus, all stereoisomers (for example, geometric isomers, optical isomers and the like) of the present compounds (including those of the salts, and solvates of the compounds), such as those which may exist due to asymmetric carbons on various substituents, including enantiomeric forms (which may exist even in the absence of asymmetric carbons), rotameric forms, atropisomers, and diastereomeric forms, are contemplated.

Diasteromeric mixtures can be separated into their individual diastereomers on the basis of their physical chemical differences by methods well known to those skilled in the art, such as, for example, by chromatography and/or fractional crystallization. Enantiomers can be separated by converting the enantiomeric mixture into a diasteromeric mixture by reaction with an appropriate optically active compound (e.g., chiral auxiliary such as a chiral alcohol or Mosher's acid chloride), separating the diastereomers and converting (e.g., hydrolyzing) the individual diastereomers to the corresponding pure enantiomers. Also, some of the compounds of Formula (1) may be atropisomers and are considered as part of this disclosure. Stereoisomers can also be separated by use of chiral HPLC.

Some of the compounds exist as tautomers. Tautomers are in equilibrium with one another. For example, amide containing compounds may exist in equilibrium with imidic acid tautomers. Regardless of which tautomer is shown and regardless of the nature of the equilibrium among tautomers, the compounds are understood by one of ordinary skill in the art to comprise both amide and imidic acid tautomers. Thus, the amide containing compounds are understood to include their imidic acid tautomers. Likewise, the imidic acid containing compounds are understood to include their amide tautomers.

A "solvate" refers to an association or complex of one or more solvent molecules and a compound of the disclosure. Examples of solvents that form solvates include, but are not limited to, water, isopropanol, ethanol, methanol, dimethylsulfoxide, ethylacetate, acetic acid and ethanolamine.

Provided herein is a compound of formula (I) 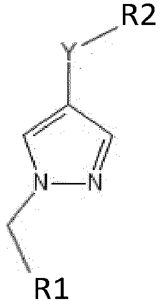 for use in the treatment and/or prevention of a condition associated with hypermineralization, wherein:
R1 is independently alkyl, aryl, cycloalkyl, heteroaryl, or heterocyclyl, optionally substituted;
R2 is nothing or independently cycloalkyl, heteroaryl, or heterocyclyl, optionally substituted; and
Y is NH, NH2, CH2, O, alkyl, alkenyl, alkynyl, S, sulfonamide, inverse amide (e.g. -NH-CO-), heterocycle or 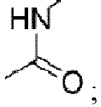
or a pharmaceutically acceptable salt, stereoisomer, mixture of stereoisomers, solvate, or tautomer thereof.
Preferably, the compound of formula I is for use in the treatment and/or prevention of a condition associated with hypermineralization,

In certain aspects, the alkyl, aryl, cycloalkyl, heteroaryl, or heterocyclyl, of R1 and/or R2 are optionally substituted with one or more aryl, alkyl, alkenyl, alkynyl, haloalkyl, cycloalkyl, aryl or heteroaryl In certain aspects, these one or more aryl, alkyl, alkenyl, alkynyl, haloalkyl, cycloalkyl, aryl or heteroarylmay also be substituted.

In certain aspects, R1 is independently selected from 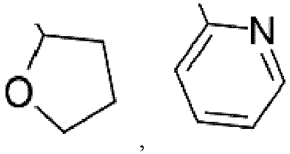 or 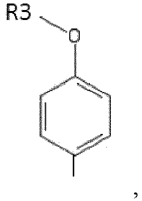 wherein R3 is an alkyl, preferably a C1-C6 alkyl.

In certain aspects, R2 is independently selected from

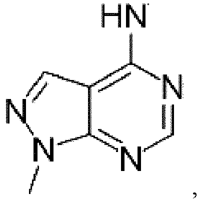

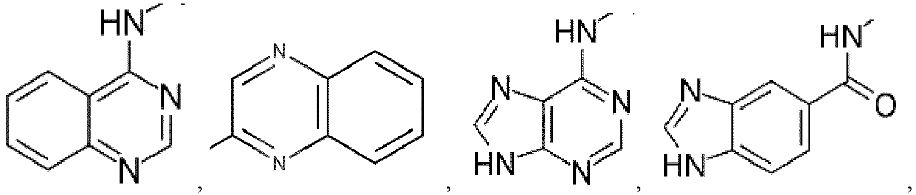

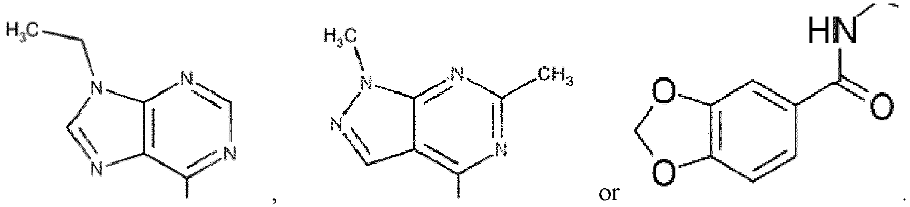

In certain aspects, the compound of the invention is selected from the following non-limiting examples of compounds: 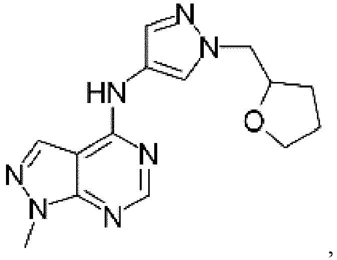 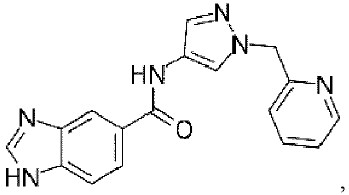 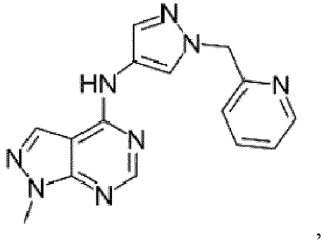 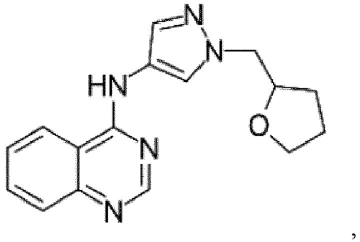 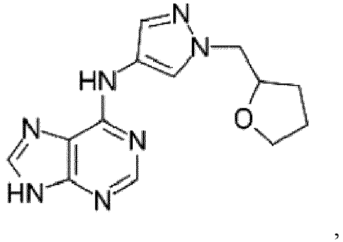 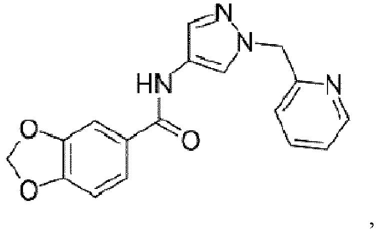 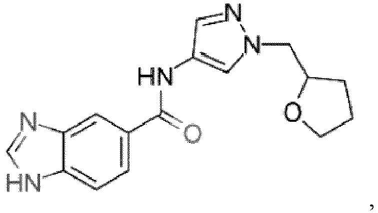 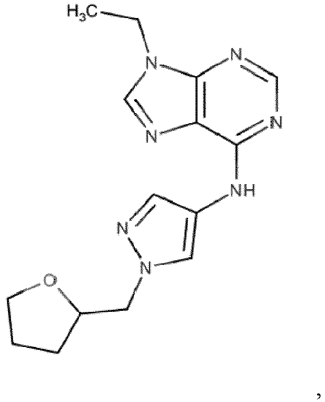 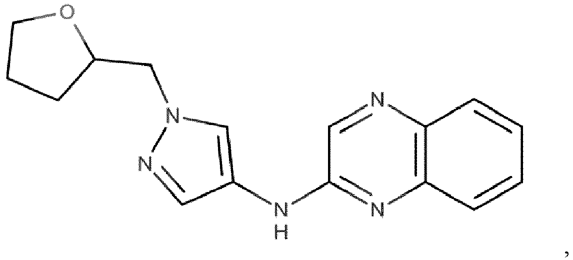 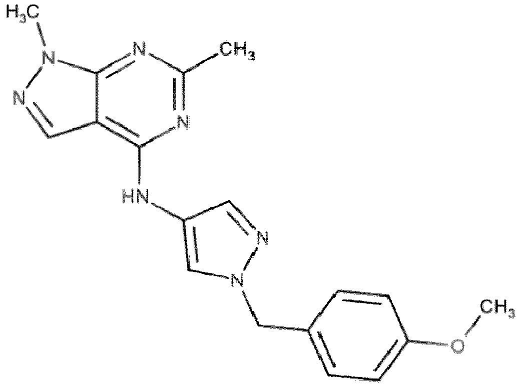 and

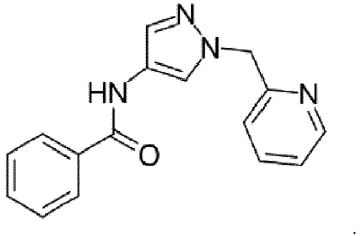

Preferably, the compound of formula (I) enhances the activity and/or expression of the cystathionine-gamma-lyase enzyme (CSE). The activity and/or expression enhancement results in an increased cellular H₂S production, a decrease in IL-6 secretion and an improvement of one or more conditions associated with hypermineralization. The one or more condition(s) associated with hypermineralization is/are selected from the group comprising heterotopic calcification, osteoarthritis, medical vascular calcification, and ankylosis. Non-limiting examples of heterotopic calcification conditions, either following trauma or surgery or due to aging, comprise any soft tissue calcifications such as, e.g., cartilage, tendon, muscle, or skin calcifications.

While it is possible for the active ingredients to be administered alone it may be preferable to present them as pharmaceutical compositions. The pharmaceutical compositions, which can be both for veterinary and for human use, comprise at least one therapeutically effective amount of a compound of formula I, a pharmaceutically acceptable salt, stereoisomer, mixture of stereoisomers, solvate, or tautomer thereof, as defined herein, together with one or more acceptable carriers therefor and optionally other therapeutic ingredients. The carrier(s) must be "acceptable" in the sense of being compatible with the other ingredients of the composition and physiologically innocuous to the recipient thereof.

The term "therapeutically effective amount" as used herein means an amount of a compound of formula I, a pharmaceutically acceptable salt, stereoisomer, mixture of stereoisomers, solvate, or tautomer thereof (hereafter "the drug"), as defined herein, high enough to significantly positively modify the symptoms and/or condition to be treated, but low enough to avoid serious side effects (at a reasonable risk/benefit ratio), within the scope of sound medical judgment.
The therapeutically effective amount of the drug as defined herein is selected in accordance with a variety of factors including type, species, age, weight, sex and medical condition of the patient; the severity of the condition to be treated; the route of administration; the renal and hepatic function of the patient. A physician of ordinary skill in the art can readily determine and prescribe the effective amount of the drug required to prevent, counter or arrest the progress of the cancer.

The pharmaceutical compositions can be administered by a number of routes including, but not limited to, oral, intravenous, intraperitoneal, intramuscular, transdermal, subcutaneous, topical, sublingual, or rectal means.

The pharmaceutical compositions include those suitable for the foregoing administration routes. The pharmaceutical compositions may conveniently be presented in unit dosage form and may be prepared by any of the methods well known in the art of pharmacy. Techniques and compositions generally are found in Remington's Pharmaceutical Sciences (Mack Publishing Co., Easton, PA). Such methods include the step of bringing into association the active ingredient, i.e. a compound of formula I, a pharmaceutically acceptable salt, stereoisomer, mixture of stereoisomers, solvate, or tautomer thereof, with inactive ingredients (e.g., a carrier, pharmaceutical excipient, etc.) which constitutes one or more accessory ingredients. In general, the compositions are prepared by uniformly and intimately bringing into association the active ingredient with liquid carriers or finely divided solid carriers or both, and then, if necessary, shaping the product.
In certain aspects, compositions suitable for oral administration are presented as discrete units such as capsules, cachets or tablets each containing a predetermined amount of the active ingredient.

The administration of the pharmaceutical compositions may be designed or formulated so as to result in sequential exposures to the compound of the invention over a certain time period, for example, hours, days, weeks, months or years. This may be accomplished, for example, by repeated administrations of a composition or by a sustained or controlled release delivery system in which the inhibitor is delivered over a prolonged period without repeated administrations. Administration of the compositions using such a delivery system may be, for example, by oral dosage forms, bolus injections, transdermal patches or subcutaneous implants. Maintaining a substantially constant concentration of the composition may be preferred in some cases.
Other delivery systems suitable include, but are not limited to, time-release, delayed release, sustained release, or controlled release delivery systems. Such systems may avoid repeated administrations in many cases, increasing convenience to the subject and the physician. Many types of release delivery systems are available and known to those of ordinary skill in the art. They include, for example, polymer-based systems such as polylactic and/or polyglycolic acids, polyanhydrides, polycaprolactones, copolyoxalates, polyesteramides, polyorthoesters, polyhydroxybutyric acid, and/or combinations of these. Microcapsules of the foregoing polymers containing nucleic acids are described in, for example, U.S. Patent No. 5,075,109. Other examples include nonpolymer systems that are lipid-based including sterols such as cholesterol, cholesterol esters, and fatty acids or neutral fats such as mono-, di- and triglycerides; hydrogel release systems; liposome-based systems; phospholipid based-systems; silastic systems; peptide-based systems; wax coatings; compressed tablets using conventional binders and excipients; or partially fused implants. Specific examples include, but are not limited to, diffusional systems in which an active component controls the release rate (for example, as described in U.S. Patent Nos. 3,832,253, 3,854,480, 5,133,974 and 5,407,686). The composition may be formulated as, for example, microspheres, hydrogels, polymeric reservoirs, cholesterol matrices, or polymeric systems. The system may allow sustained or controlled release of the composition to occur, for example, through control of the diffusion or erosion/degradation rate of the formulation containing the compound of the invention. In addition, a pump-based hardware delivery system may be used for delivery.
In certain aspects, the pharmaceutical compositions include one or more compounds of formula I of the invention together with one or more pharmaceutically acceptable carriers or excipients and optionally other therapeutic agent(s). Pharmaceutical compositions containing the active ingredient may be in any form suitable for the intended method of administration. When used for oral use for example, tablets, troches, lozenges, aqueous or oil suspensions, dispersible powders or granules, emulsions, hard or soft capsules, syrups or elixirs may be prepared. Compositions intended for oral use may be prepared according to any method known to the art for the manufacture of pharmaceutical compositions and such compositions may contain one or more agents including sweetening agents, flavoring agents, coloring agents and preserving agents, in order to provide a palatable preparation. Tablets containing the active ingredient in admixture with non-toxic pharmaceutically acceptable excipient which are suitable for manufacture of tablets are acceptable. These excipients may be, for example, inert diluents, such as calcium or sodium carbonate, lactose, lactose monohydrate, croscarmellose sodium, povidone, calcium or sodium phosphate; granulating and disintegrating agents, such as maize starch, or alginic acid; binding agents, such as cellulose, microcrystalline cellulose, starch, gelatin or acacia; and lubricating agents, such as magnesium stearate, stearic acid or talc. Tablets may be uncoated or may be coated by known techniques including microencapsulation to delay disintegration and adsorption in the gastrointestinal tract and thereby provide a sustained action over a longer period. For example, a time delay material such as glyceryl monostearate or glyceryl distearate alone or with a wax may be employed.
The amount of active ingredient that is combined with the inactive ingredients to produce a dosage form will vary depending upon the host treated and the particular mode of administration. For example, in some aspects, a dosage form for oral administration to humans contains approximately 1 to 1000 mg of active material formulated with an appropriate and convenient amount of carrier material (e.g., inactive ingredient or excipient material). In certain embodiments, the carrier material varies from about 5 to about 95% of the total compositions (weight: weight). In some aspects, the pharmaceutical compositions described herein contain about 1 to 800 mg, 1 to 600 mg, 1 to 400 mg, 1 to 200 mg, 1 to 100 mg or 1 to 50 mg of the compound of Formula I, or stereoisomer, mixture of stereoisomers, solvate, or tautomer thereof. In some aspects, the pharmaceutical compositions described herein contain not more than about 400 mg of the compound of Formula I. In some embodiments, the pharmaceutical compositions described herein contain about 100 mg of the compound of Formula I, or a pharmaceutically acceptable salt thereof.

It should be understood that in addition to the ingredients particularly mentioned above the compositions disclosed herein may include other agents conventional in the art having regard to the type of composition in question, for example those suitable for oral administration may include flavoring agents.
Preferably, pharmaceutical compositions of the invention are for use in the treatment and/or prevention of one or more conditions associated with hypermineralization. These conditions have been defined above.

In some aspects, the pharmaceutical compositions of the invention further comprise other therapeutic agents (e.g. corticosteroids) and/or are administered in association with a treatment selected from the group comprising surgery, intra-articular injection of corticosteroids.

Also provided is a method of treatment and/or prevention of a condition associated with hypermineralization, said method comprising administering a compound of the invention as therapeutic agent or a pharmaceutical composition of the invention to a subject.

As used herein, the term "treatment" or "treating" means any administration of a composition, pharmaceutical composition, therapeutic agent, compound, *etc...* of the disclosure to a subject for the purpose of:
(i) inhibiting the disease, that is, arresting the development of clinical symptoms; and/or
(ii) relieving the disease, that is, causing the regression of clinical symptoms.

As used herein, the term "prevention" or "preventing" means any administration of a composition, pharmaceutical composition, therapeutic agent, compound, *etc...* of the disclosure to a subject for the purpose of preventing the disease, that is, causing the clinical symptoms of the disease not to develop.

In the context of the present invention, the disease is one or more conditions associated with hypermineralization as defined herein.

The administration routes are, preferably, chosen among those described herein.

The terms "inhibit," "inhibiting," and "inhibition" refer to the slowing, halting, or reversing the growth or progression of a disease, infection, condition, or group of cells. The inhibition can be greater than about 20%, 40%, 60%, 80%, 90%, 95%, or 99%, for example, compared to the growth or progression that occurs in the absence of the treatment or contacting.

Alternatively, the method of treatment and/or prevention can be associated with a treatment selected from the group comprising surgery and intra-articular injection of corticosteroids.

"Administering", as it applies in the present invention, refers to contact of a therapeutically effective amount of an inhibitor of the invention, to the subject.

As used herein the terms " subject", or " patient" are well-recognized in the art, and, are used interchangeably herein to refer to a mammal, including dog, cat, rat, mouse, monkey, cow, horse, goat, sheep, pig, camel, and, most preferably, a human. In some aspects, the subject is a subject in need of treatment or a subject suffering from one or more conditions associated with hypermineralization or a subject that might be at risk of suffering from one or more conditions associated with hypermineralization. However, in other aspects, the subject can be a normal subject. The term does not denote a particular age or sex. Thus, adult and newborn subjects, whether male or female, are intended to be covered.

The present invention also contemplates a method for inhibiting, regulating and/or preventing chondrocytes calcification, comprising administering a compound of the invention or a pharmaceutical composition of the invention to a subject. The inhibition can be greater than about 20%, 40%, 60%, 80%, 90%, 95%, or 99%, for example, compared to the growth or progression that occurs in the absence of the treatment or contacting.

The present invention further contemplates a method for inhibiting, regulating and/or preventing cartilage degradation, comprising administering a compound of the invention or a pharmaceutical composition of the invention to a subject. The inhibition can be greater than about 20%, 40%, 60%, 80%, 90%, 95%, or 99%, for example, compared to the growth or progression that occurs in the absence of the treatment or contacting.

Those skilled in the art will appreciate that the invention described herein is susceptible to variations and modifications other than those specifically described. It is to be understood that the invention includes all such variations and modifications without departing from the spirit or essential characteristics thereof. The invention also includes all of the steps, features, compositions and compounds referred to or indicated in this specification, individually or collectively, and any and all combinations or any two or more of said steps or features. The present disclosure is therefore to be considered as in all aspects illustrated and not restrictive, the scope of the invention being indicated by the appended Claims, and all changes which come within the meaning and range of equivalency are intended to be embraced therein.

Various references are cited throughout this Specification, each of which is incorporated herein by reference in its entirety. The foregoing description will be more fully understood with reference to the following Examples.

### EXAMPLES

### Material and Methods

### Mice

*Cse* knock-out (*Cse* KO) (Ishii et al. 2010), *Cse* transgenic (*Cse* tg; under review, J. Mitchell et al.), both In a C57BL/6 background, and *Cbs* knock-out (*Cbs* KO) (Akahoshi et al. 2008) in the C3H/HeJ background, were used. For controls, age, sex and background matched wild type (WT) mice were used.

### Induction of knee heterotopic calcification

For spontaneous murine knee HC and subsequent OA, 61-64 weeks old female WT and *Cse* KO mice were studied. For surgically-induced murine knee HC and subsequent OA, 12 weeks old female WT, *Cse* KO and *Cse* tg mice were used. Mice were anesthetized via continuous isoflurane inhalation. Knee joint instability was induced surgically by partial medial menisectomy (MNX) of the right knee, whereas the contralateral knee was sham-operated as control (Kamekura et al. 2005). At the end of both experiments, mice were sacrificed via CO₂ inhalation and knees dissected and fixed in 10% formalin for further analysis.

### Induction of Achilles tendon heterotopic calcification

For spontaneous murine Achilles tendon HC, 35 weeks old female *Cse* KO and WT mice were studied. For surgically-induced murine Achilles tendon HC, surgical technique was performed as described recently by Michel et al.(Michel et al. 2020). Animals were anaesthetized with isoflurane 2.5 vol. % (Forene; AbbVie, Ludwigshafen, Germany). The left hindlimb of the animal was fixed in a standardized dorsal extension of the upper ankle joint. A dorsomedial skin incision was made and a full-thickness tenotomy was performed approximately 3 mm proximal to the calcaneus followed by insertion of a tibiofibular Prolene 5-0 cerclage to achieve postoperative immobilization of the upper ankle joint. The Achilles tendon was then readapted with 2 independent PDS 7-0 core sutures (4 strands crossing the tenotomy site). Animals were used for histological analysis 1, 2, 4, 6 and 16 weeks after surgery, fixated Achilles tendons (4% paraformaldehyde overnight) were decalcified for 6 weeks and serial cross sections were cut at a thickness of 5 µm at the colourmarked tenotomy site for further immunohistochemical investigations.

### MicroCT- scan

For knee microCT-scan, samples were dissected and fixed in formol. Images were acquired using a SkyScan 1076 X-ray µCT scanning system (SkyScan, Belgium) with the following parameters: 18 µm resolution, 60 kV, 167 µA, 0.4° rotation step over 360°, 0.5 mm aluminum filter, 1180 ms exposure time. 3D reconstruction was performed using NRecon V1.6.6.0 (Skyscan, Belgium) with the following parameters: grey values=0.0000-0.105867, ring artefact reduction=3, beam hardening correction=40%. Quantitative analyses of knee heterotopic ossification (HC), in particular HC Volume (mm³) and HC crystal content (µg), were conducted using CTAnalyzer V.1.10 (SkyScan, Belgium).

For Achilles tendon microCT-scan, lower limbs were dissected and fixed in formol. Images were acquired using a SkyScan 1076 X-ray µCT scanning system (SkyScan, Belgium) with the following parameters: 9 µm resolution, 45 kV, 556 µA, 0.5° rotation step over 360°, 0.2 mm aluminum filter, 780 ms exposure time. 3D reconstruction was performed using NRecon V1.6.6.0 (Skyscan, Belgium) with the following parameters: grey values=0.0000-0.105867, ring artefact reduction=10, beam hardening correction=20%. Quantitative analyses of tendon heterotopic calcification (HC), in particular HC Volume (mm³), HC crystal content (µg), and HC trabecular thickness Tb.Th (mm) were conducted using CTAnalyzer V.1.10 (SkyScan, Belgium).

### Mouse knee histology

After microCT-scan image acquisition, knees were decalcified in 5% formic acid for 10 days, dehydrated, and embedded in paraffin. Sagittal knee sections (6 µm) were stained with Safranin-O and counterstained with fast green/iron hematoxylin. For the surgically-induced HC model, 3 sections per mouse, spaced 70 µM apart, were taken through the medial compartment. For the spontaneous HC model, 3 sections per mouse, spaced 70 µM apart, were taken through the whole joint. Histological scorings (cartilage damage and Safranin-O loss) were assessed using the OARSI score (Pritzker et al. 2006), by two independent observers blinded with regard to the mice groups.

### Murine chondrocyte and tenocytes isolation and induction of calcification

Chondrocytes were isolated from 6 days old mice (*Cse* KO, *Cse* tg, *Cbs* KO and corresponding WT mice) as described previously (Nasi, So, et al. 2016). Tenocytes were isolated from 6 to 8 weeks old mice (*Cse* KO and WT) and by double digestion in Collagenase IV NB standard grade (Witec AG). Chondrocytes were amplified in DMEM with 10% FBS and 1% Penicillin Streptomycin and used at P1. Tenocytes were amplified in αDMEM with 10% FBS, 0.2 mM L-ascorbic acid, 2-phosphate and 1% Penicillin Streptomycin and used at P1. For crystal formation analysis, cells were cultured for 14 days in calcification medium (BGJb (Gibco) medium with 10% FBS, 0.2 mM L-ascorbic acid, 2-phosphate, 20mM β-glycerol phosphate and 1% Penicillin Streptomycin). Medium and stimulations were refreshed every 3 days of culture. Alternatively, in some experiments, cells were cultured for 24h in medium supplemented with secondary calciprotein particles (CPP, final concentration equivalent to 100 mg/mL calcium) to induce calcification as previously described (Aghagolzadeh et al. 2017). Cells were treated with sodium thiosulfate (STS 25Mm, Sigma), homocysteine (Hcy 25 or 100µM, Sigma), or the CSE activator (SU004 50µM) where indicated.

### Crystal detection in chondrocyte and tenocytes cultures

After incubation in calcification media, supernatant was collected for ELISA and LDH measurement, and cell monolayer was washed in PBS, fixed in 10% formol and crystal deposition analyzed through Alizarin red staining and quantified as previously described (Gregory et al. 2004). In addition, for some experiments, quantification of calcium content in the cell monolayer was performed by the QuantiChrom^{™} Calcium Assay Kit (BioAssay Systems) as specified by manufacturer's protocols, and absorbance was read at 612nm using the Spectramax M5e plate reader (Molecular Devices).

### Calcium phosphate crystals stimulation

Hydroxyapatite (HA) crystals were synthesized and characterized as previously described (Prudhommeaux et al. 1996). HA crystals were sterilized by gamma-radiation and pyrogen-free (≤ 0.01 EU/10 mg- by Limulus amebocyte cell lysate assay). Prior to experimentation, crystals were resuspended in sterile PBS and sonicated for 5 min.

### Alkaline phosphatase activity

Chondrocytes supernatant was removed and alkaline phosphatase (*Alpl*) activity was measured in cell lysate using a p-Nitrophenyl Phosphate assay (Alkaline Phosphatase Assay Kit, Abcam, ab83369). The manufacturer's protocols were explicitly followed, and absorbance was read at 405 nm at the Spectramax M5e plate reader.

### Real time PCR analysis

RNA was extracted (RNA Clean & Concentrator5, Zymoresearch), reverse transcribed (Superscript II, Invitrogen), and quantitative Real Time PCR (qRT-PCR) with gene specific primers using the LightCycler480^{®}system (Roche Applied Science) was performed. Murine data was normalized against *Tbp* and *Gapdh* references genes, with fold induction of transcripts calculated against control cells.

### Human cartilage experiments

Human OA articular cartilage was obtained from 15 patients undergoing knee joint replacement (Kellgren-Lawrence score from 1 to 4, 64.69 years ± 10.58 years) from the Otto-von-Guericke University (Magdeburg). Full thickness cartilage pieces were resected from the main loading area of the medial compartment of both tibia and femur. Cartilage tissue was fixed in freshly prepared 4% paraformaldehyde for immunohistochemical and calcification analysis.

For HA crystal stimulation experiment, cartilage from tibial plateau and femoral condyles from 4 OA patients (mean age 72±10 years) undergoing total knee replacement (Kellgren and Laurence (KL) score=4), obtained from the Orthopedics Department (DAL, CHUV, Lausanne-CH) was used. 6 mm diameter disks (3 disks/patient) were dissected from macroscopically intact cartilage using a dermal punch. In order to match for location across treatment groups, each disk was divided in two equal parts, and each half was stimulated or not with 500µg/ml of HA crystals for 24h in individual 96 wells, coated with **Poly** (2-hydroxyethyl methacrylate) in culture medium (DMEM+1% Penicillin Streptomycin+50ug/ml L-ascorbic acid 2-phosphate). At the end of the incubation period, cartilage was recovered for CSE immunohistochemistry.

For chondrocyte isolation, cartilage from tibial plateau and femoral condyles from 3 OA patients (mean age 70±8 years) undergoing total knee replacement (Kellgren and Laurence (KL) score=4), obtained from the Orthopedics Department (DAL, CHUV, Lausanne-CH) was used.

### Human chondrocyte isolation and induction of calcification

Chondrocytes were isolated from cartilage pieces incubated overnight in the digestion enzyme Liberase TM (Roche). For chondrocyte crystal formation analysis, cells were cultured for 24h in medium supplemented with secondary calciprotein particles (CPP, final concentration equivalent to 100 mg/mL calcium) to induce calcification as described previously (Aghagolzadeh et al. 2017).

### Human cartilage histology and quantification of calcification

Paraffin sections (4µm) were cut and were stained with von Kossa/Safranin-Orange staining (Sigma). OARSI scoring (from 0 to 5) was performed and cartilage samples were grouped into low (K/L 1-2 and OARSI 2-3), medium (K/L 3 and OARSI 3-4) and high (K/L 4 and OARSI 5) OA grade. Quantitative measurement of the areas of total cartilage mineralization were performed using ImageJ (NIH Image), by setting the upper and lower bounds of the threshold utility to a specified level for all samples. These areas were then used for calculating the percentage of calcification in relation to the surrounding whole cartilage area. The percentage of calcification was determined by dividing the area of mineralization by the whole area of cartilage.

### Immunohistochemical analysis

Human CSE expression was evaluated using an anti-CSE rabbit polyclonal antibody (Sigma, HPA-023300). Murine CSE expression was evaluated using an anti-CSE rabbit polyclonal antibody (Proteintech, CSE 12217-1-AP). Human and murine CBS expression was evaluated using an anti-CBS goat polyclonal antibody (Santa Cruz, SC46830). Apoptotic chondrocytes were detected using the ApopTag plus Peroxidase In situ Kit (Millipore) as previously described (Nasi et al. 2014). All stainings were performed on paraffin sections. For cartilage samples, the percentage of positive cells over the total number of cells was quantified in three different fields for each sample and the mean plotted in the graphs. For tendon samples, an arbitrary score from 0=no *Cse* expression to 4=massive *Cse* expression was given to three fields for each sample and the mean values plotted in the graphs.

### H₂S measurements

Chondrocytes from *Cse* KO, *Cse* tg, *Cbs* KO and corresponding WT mice were resuspended in fluorescence-activated cell sorting (FACS) buffer (5%FCS, 5mM EDTA in PBS). For each condition, 10⁶ cells were incubated with the H₂S fluorescent probe P3 (10µM) (Singha et al. 2015) and after two minutes FACS analysis was performed on a LSRII SORP cytometer (BD Biosciences) with an UV laser. FACS Diva (BD Biosciences) and FlowJoX (Tree Star) software were used for data processing.

### IL-6 quantification

At the reported time points, cell supernatants were collected and assayed using murine or human IL-6 ELISA kit (eBioscience). The manufacturer's protocols were explicitly followed, and absorbance was read at 450 nm and 570 nm using the Spectramax M5e plate reader.

### LDH measurement

LDH in supernatant was measured using CytoTox-ONE^{™} Homogeneous Membrane Integrity Assay (Promega) according to the manufacturer's instructions. LDH release (%) was calculated by using the following formula. LDH release (%) = [(value in sample) - (background)] / [(value in Triton X-100-treated sample) - (background)] x100.

### Ethics statements

Tendon experiments in mice were carried out with permission of the local animal rights protection authorities (approval number: 84-02.04.2015.A310) in accordance with the National Institutes of Health guidelines for the use of laboratory animals. Animal care and treatment were provided in accordance with institutional guidelines. The animals were housed in groups of 2-5 at a constant room temperature of 21°C, at an atmospheric humidity of 65%, and under a 12-h light/dark cycle. They had free access to tap water and standard feed.Experiments in mice were performed in strict accordance to the Swiss Federal Regulations. The protocol was approved by the *"Service de la consommation et des affaires veterinaires du Canton de Vaud",* Switzerland. All efforts were made to minimize suffering. Human samples were obtained with the approval of the Centre Hospitalier Universitaire Vaudois ethical committee or by the institutional Review Board of the Faculty of Medicine of the Otto-von-Guericke University (IRB No 23/16), and written informed consent of patients was obtained.

### Statistical analysis

For *in vitro* experiments, values represent means±SD of triplicates from one representative experiment of three independent experiments. For *in vivo* experiments, at least 5 mice per group were used. Data was analyzed with GraphPad Prism software (GraphPad software), San Diego, CA. Variation between data sets was evaluated using the Student's t test or One-way or Two-way ANOVA test, where appropriate. Correlations were evaluated with Pearson's r correlation. Differences and correlation were considered statistically significant at **p<0.05,* ***p*<0.01, ****p*<0.001, *****p*<0.0001.

### Chemical syntheses

All solvents were purchased from Sigma-Aldrich and used as received. All NMR spectra were recorded on a Bruker AVANCE III HD 500 One Bay spectrometer with a magnetic field of 11.75 T and a 5 mm SmartProbe BB(F)-H-D. For ¹H NMR spectra, a frequency of 500 MHz resulted. Chemical shifts are reported in ppm from tetramethylsilane or solvent residual peaks as internal standard. Data are reported as follows: chemical shift, multiplicity (s = singlet, d = doublet, t = triplet, q = quartet, br = broad, m = multiplet), coupling constants (Hz), integration. For ¹³C NMR spectra, a frequency of 125 MHz resulted. Purity was assayed by HPLC (Interchim Strategy C-18 column, 4.6mm × 250 mm) with a gradient of 5-100% methanol (0.2 % acetic acid) in 0.2% aqueous acetic acid with UV detection at λ = 254 nm. All final compounds were obtained with >95% purity.

| **Supplier** | **Cat.No** | **Name** | CAS **No.** |
|---|---|---|---|
| Fluorochem | 026197 | 4-Nitro-1H-pyrazole | 2075-46-9 |
| Sigma-Aldrich | 185396 | Tetrahydrofuryl alcohol (rac.) | 97-99-4 |
| Fluka | 93092 | Triphenylphosphine | 603-35-0 |
| Sigma-Aldrich | 11625 | Di-tert-butyl azodicarboxylate | 870-50-8 |
| Sigma-Aldrich | 520829 | Palladium on carbon 10 % | -- |
| Sigma-Aldrich | 491047 | 2-(Bromomethyl)pyridine hydrobromide | 31106-82-8 |
| Sigma-Aldrich | 700096 | 4-Chloroquinazoline | 5190-68-1 |
| Fluorochem | 067568 | 4-Chloro-1-methyl-1H-pyrazolo[3,4-d]pyrimidine | 23000-43-3 |
| Fluorochem | 032518 | 6-Chloropurine | 87-42-3 |
| Fluorochem | 023926 | 1-[bis(Dimethylamin)methylen]-1H-1,2,3-triazol[4,5-b]pyridinium- 3-oxid-hexafluorophosphat (HATU) | 148893-10-1 |
| TCI | P0459 | Piperonylic acid | 94-53-1 |
| Sigma-Aldrich | D125806 | *N*,*N*-diisopropylethylamine (DIPEA) | 7087-68-5 |
| TCI | B1763 | 5-Benzimidazolecarboxylic acid | 15788-16-6 |
| Sigma-Aldrich | 242381 | Benzoic acid | 65-85-0 |
| Fluorochem | 043654 | 4-Chloro-7H-pyrrolo(2,3,D)pyrimidine | 3680-69-1 |

### Synthesis schemes

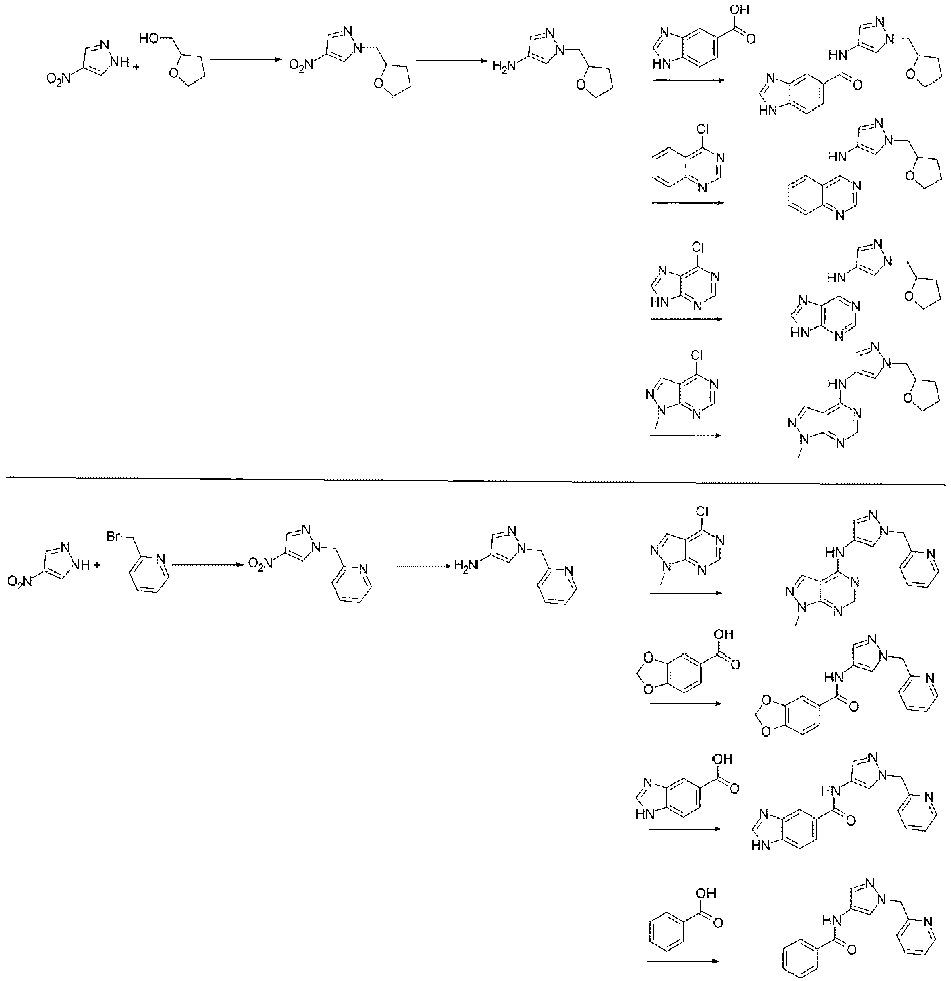

### Synthesis of C5 and C6 and derivatives thereof

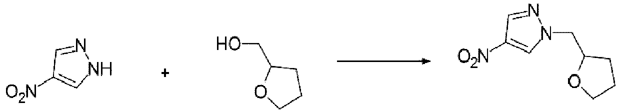

### 4-nitro-1-[(oxolan-2-yl)methyl]-1H-pyrazole, ZHAWOC8589 (Compound 1)

To a solution of 4-nitro-1H-pyrazole (500 mg, 4.42 mmol, 1 eq.), tetrahydrofuryl alcohol (rac.) (452 mg, 4.42 mmol, 1.0 eq.), and triphenylphosphine (1392 mg, 5.31 mmol, 1.2 eq.) in 22 ml of dry tetrahydrofuran under argon atmosphere at 20 °C was added di-tert-butyl azodicarboxylate (1323 mg, 5.75 mmol, 1.3 eq.). The reaction mixture was concentrated after 4 h and the crude product purified by silica gel flash chromatography using a gradient from cyclohexane to 30 % ethyl acetate in cyclohexane to afford 4-nitro-1-[(oxolan-2-yl)methyl]-1H-pyrazole (870 mg, 4.42 mmol, 100 %).
¹H NMR (500 MHz, DMSO-*d₆*) δ 8.82 (d, *J* = 0.6 Hz, 1H), 8.28 (d, *J* = 0.6 Hz, 1H), 4.30 - 4.15 (m, 3H), 3.75 (dt, *J* = 8.1, 6.8 Hz, 1H), 3.64 (dt, *J* = 8.1, 6.8 Hz, 1H), 2.02 - 1.92 (m, 1H), 1.87 - 1.73 (m, 2H), 1.66 - 1.56 (m, 1H).
¹³C NMR (126 MHz, DMSO-*d₆*) δ 136.0, 135.3, 131.4, 76.8, 67.8, 56.5, 28.6, 25.4.

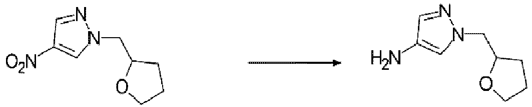

### 4-amino-1-[(oxolan-2-yl)methyl]-1H-pyrazole , ZHAWOC8590 (Compound 2)

A suspension of 4-nitro-1-[(oxolan-2-yl)methyl]-1H-pyrazole (ZHAWOC8589) (870 mg, 4.42 mmol) and 10 % palladium on carbon (290 mg) in methanol (22 mL) was placed under a hydrogen atmosphere by briefly evacuating the flask, then flushing with pure hydrogen from a balloon. The black suspension was stirred for 1 h at 20 °C, filtered through a pad of celite and washed with additional methanol. Concentration of the filtrate yielded 4-amino-1-[(oxolan-2-yl)methyl]-1H-pyrazole (690 mg, 4.13 mmol, 94 %).
¹H NMR (500 MHz, DMSO-*d₆*) δ 7.02 (d, *J* = 0.9 Hz, 1H), 6.89 (d, *J* = 0.9 Hz, 1H), 4.09 - 4.02 (m, 1H), 3.99 - 3.90 (m, 2H), 3.80 (br, 2H), 3.75 - 3.69 (m, 1H), 3.64 - 3.58 (m, 1H), 1.91 - 1.66 (m, 3H), 1.59 - 1.48 (m, 1H).
¹³C NMR (126 MHz, DMSO-*d₆*) δ 131.2, 129.6, 117.5, 77.9, 67.8, 55.6, 28.7, 25.4.

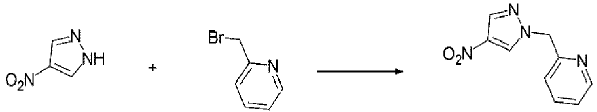

### [(4-nitro-1H-pyrazol-l-yl)methyl]pyridine, ZHAWOC8591 (Compound 3)

To a solution of 4-nitro-1H-pyrazole (500 mg, 4.42 mmol, 1.0 eq.) in DMF (26 mL) was added K₂CO₃ (733 mg, 5.31 mmol, 1.2 eq.) and 2-(bromomethyl)pyridine hydrobromide (1118 mg, 4.42 mmol, 1.0 eq.). The reaction mixture was stirred overnight at room temperature, diluted with ethyl acetate and washed twice with 1:1 H₂O / brine. The organic extract was dried over Na₂SO₄ and concentrated in vacuo. The crude product was purified by silica gel flash chromatography using a gradient from cyclohexane to 50 % ethyl acetate in cyclohexane to afford [(4-nitro-1H-pyrazol-1-yl)methyl]pyridine (690 mg, 3.38 mmol, 76 %).
¹H NMR (500 MHz, CDCl₃) δ 8.62 (d, *J=* 4.7 Hz, 1H), 8.34 (s, 1H), 8.11 (s, 1H), 7.74 (ddd, *J* = 7.7, 7.7, 1.7 Hz, 1H), 7.37 - 7.20 (m, 2H), 5.44 (s, 2H).
¹³C NMR (126 MHz, CDCl₃) δ 153.8, 150.1, 137.4, 136.2, 136.1, 129.3, 123.7, 122.6, 58.7.

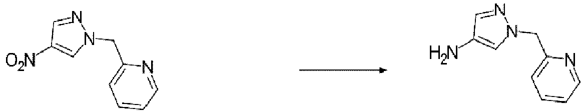

### [(pyridin-2-yl)methyl]-1H-pyrazol-4-amine, ZHAWOC8592 (Compound 4)

The [(4-nitro-1H-pyrazol-1-yl)methyl]pyridine (690 mg, 3.38 mmol) was reduced to [(pyridin-2-yl)methyl]-1H-pyrazol-4-amine (520 mg, 2.99 mmol, 88 %) similar to the procedure of 4-amino-1-[(oxolan-2-yl)methyl]-1H-pyrazole (ZHAWOC8590) to afford [(pyridin-2-yl)methyl]-1H-pyrazol-4-amine (520 mg, 2.99 mmol, 88 %).
¹H NMR (500 MHz, DMSO-*d₆*) δ 8.52 (d, *J* = 4.7 Hz, 1H), 7.74 (ddd, *J* = 7.7, 7.7, 1.8 Hz, 1H), 7.28 (ddd, *J* = 7.5, 4.9, 0.6 Hz, 1H), 7.14 (s, 1H), 6.99 (s, 1H), 6.90 (d, *J* = 7.8 Hz, 1H), 5.23 (s, 2H), 3.88 (br, 2H).
¹³C NMR (126 MHz, DMSO-*d₆*) δ 158.1, 149.4, 137.4, 131.9, 130.5, 123.0, 121.7, 117.6, 57.1.

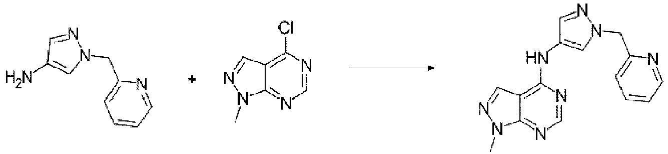

### N-{1-methyl-1H-pyrazolo[3,4-d]pyrimidin-4-yl}-1-[(pyridin-2-yl)methyl]-1H-pyrazol-4-amine, ZHAWOC8593 (Compound 5)

4-Chloro-1-methyl-1H-pyrazolo[3,4-d]pyrimidine (97 mg, 0.379 mmol, 1.0 eq.) and [(pyridin-2-yl)methyl]-1H-pyrazol-4-amine (ZHAWOC8592) (100 mg, 5.74 mmol, 1.0 eq) were dissolved in *N*,*N*-dimethylformamide (DMF) (3 ml) and then heated to 80 °C for 1 hour. The reaction mixture was allowed to cool to room temperature and then acetonitrile (10 ml) was added and the resulted suspension filtered. The crude product was purified by silica gel flash chromatography using a gradient from dichloromethane to 20 % methanol in dichloromethane to afford *N*-{1-methyl-1H-pyrazolo[3,4-d]pyrimidin-4-yl}-1-[(pyridin-2-yl)methyl]-1H-pyrazol-4-amine (116 mg, 0.379 mmol, 66 %).
¹H NMR (500 MHz, DMSO-*d₆*) δ 10.32 (s, 1H), 8.63 - 8.50 (m, 1H), 8.42 (s, 1H), 8.36 (s, 1H), 8.21 (s, 1H), 7.85 - 7.75 (m, 1H), 7.72 (s, 1H), 7.43 - 7.25 (m, 1H), 7.16 - 6.95 (m, 1H), 5.46 (s, 2H), 3.94 (s, 3H).
¹³C NMR (126 MHz, DMSO-*d₆*) δ 157.4, 156.1, 153.3, 153.1, 149.6, 137.6, 131.7, 131.5, 123.3, 122.5, 122.0, 101.0, 100.0, 57.3, 34.0.

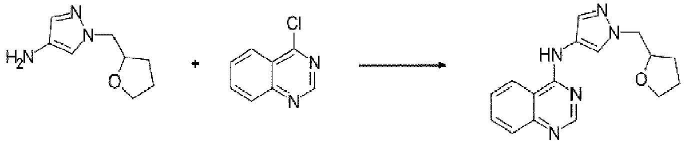

### N-{1-[(oxolan-2-yl)methyl]-1H-pyrazol-4-yl}quinazolin-4-amine ZHAWOC8598 (Compound 6)

4-chloroquinazoline (49 mg, 0.299 mmol, 1.0 eq.) and 4-amino-1-[(oxolan-2-yl)methyl]-1H-pyrazole (ZHAWOC8590) (50 mg, 0.299 mmol, 1.0 eq.) were treated similar to the procedure of *N*-{1-methyl-1H-pyrazolo[3,4-d]pyrimidin-4-yl}-1-[(pyridin-2-yl)methyl]-1H-pyrazol-4-amine (ZHAWOC8593) to yield *N*-{1-[(oxolan-2-yl)methyl]-1H-pyrazol-4-yl}quinazolin-4-amine (55 mg, 0.186 mmol, 62 %).
¹H NMR (500 MHz, DMSO-*d₆*) δ 12.15 (s, 1H), 9.01 (s, 1H), 8.99 - 8.95 (m, 1H), 8.39 (s, 1H), 8.09 - 8.03 (m, 2H), 7.98 - 7.92 (m, 1H), 7.86 - 7.80 (m, 1H), 4.30 - 4.23 (m, 1H), 4.23 - 4.13 (m, 2H), 3.77 (dd, *J* = 14.5, 7.0 Hz, 1H), 3.65 (dd, *J* = 14.3, 7.5 Hz, 1H), 2.00 - 1.90 (m, 1H), 1.85 - 1.71 (m, 2H), 1.66- 1.56 (m, 1H).
¹³C NMR (126 MHz, DMSO-*d₆*) δ 157.1, 151.6, 138.6, 136.2, 133.1, 128.9, 125.1, 124.8, 120.6, 120.1, 114.0, 77.6, 67.9, 56.1, 28.7, 25.5.

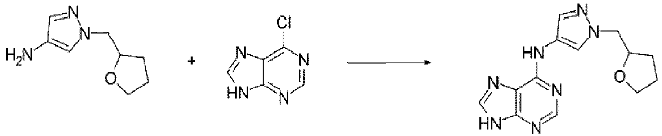

### N-{1-[(oxolan-2-yl)methyl]-1H-pyrazol-4-yl}-9H-purin-6-amine, ZHAWOC8599 (Compound 7)

6-Chloropurine (46 mg, 0.299 mmol, 1.0 eq.) and 4-amino-1-[(oxolan-2-yl)methyl]-1H-pyrazole (ZHAWOC8590) (50 mg, 0.299 mmol, 1.0 eq.) were treated similar to the procedure of *N*-{1-methyl-1H-pyrazolo[3,4-d]pyrimidin-4-yl}-1-[(pyridin-2-yl)methyl]-1H-pyrazol-4-amine (ZHAWOC8593) to yield N-{1-[(oxolan-2-yl)methyl]-1H-pyrazol-4-yl}-9H-purin-6-amine (50 mg, 0.175 mmol, 59 %).
¹H NMR (500 MHz, DMSO-*d₆*) δ 10.76 (s, 1H), 8.54 (s, 1H), 8.44 (s, 1H), 8.17 (s, 1H), 7.74 (s, 1H), 4.26 - 4.07 (m, 3H), 3.76 (dd, *J* = 14.5, 6.9 Hz, 1H), 3.64 (dd, *J* = 14.5, 7.2 Hz, 1H), 2.00 - 1.86 (m, 1H), 1.86 - 1.69 (m, 2H), 1.64 - 1.54 (m, 1H). (One protic hydrogen undergoes a deuterium exchange.)
¹³C NMR (126 MHz, DMSO-*d₆*) δ 150.6, 150.0, 149.4, 142.0, 131.6, 122.5, 121.5, 115.9, 77.7, 67.9, 56.0, 28.7, 25.5.

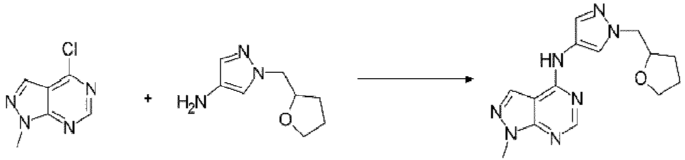

### N-{1-methyl-1H-pyrazolo[3,4-d]pyrimidin-4-yl}-1-[(oxolan-2-yl)methyl]-1H-pyrazol-4-amine, ZHAWOC8600 (Compound 8)

4-Chloro-1-methyl-1H-pyrazolo[3,4-d]pyrimidine (101 mg, 0.598 mmol, 1.0 eq.) and 4-amino-1-[(oxolan-2-yl)methyl]-1H-pyrazole (ZHAWOC8590) (100 mg, 0.598 mmol, 1.0 eq.) were treated similar to the procedure of *N*-{1-methyl-1H-pyrazolo[3,4-d]pyrimidin-4-yl}-1-[(pyridin-2-yl)methyl]-1H-pyrazol-4-amine (ZHAWOC8593) to yield *N*-{1-methyl-1H-pyrazolo[3,4-d]pyrimidin-4-yl}-1-[(oxolan-2-yl)methyl]-1H-pyrazol-4-amine (105 mg, 0.351 mmol, 59 %).
¹H NMR (500 MHz, DMSO-*d₆*) δ 11.18 (br, 1H), 8.56 - 8.36 (m, 2H), 8.16 (s, 1H), 7.71 (s, 1H), 4.28 - 4.08 (m, 3H), 3.98 (s, 3H), 3.76 (dd, *J=* 14.3, 7.0 Hz, 1H), 3.64 (dd, *J=* 14.3, 7.1 Hz, 1H), 2.04 - 1.88 (m, 1H), 1.88 - 1.69 (m, 2H), 1.69 - 1.54 (m, 1H).
¹³C NMR (126 MHz, DMSO-*d₆*) δ 152.6, 152.3, 151.1, 133.4, 132.6, 124.3, 119.8, 100.8, 77.6, 67.9, 56.1, 34.6, 28.7, 25.5.

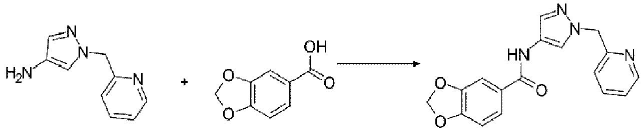

### N-{1-[(pyridin-2-yl)methyl]-1H-pyrazol-4-yl}-2H-1,3-benzodioxole-5-carboxamide, ZHAWOC8595 (Compound 9)

1-[bis(Dimethylamin)methylen]-1*H*-1,2,3-triazol[4,5-*b*]pyridinium-3-oxid-hexafluorophosphat (HATU) (172 mg, 0.451 mmol, 1.5 eq.) was added to a mixture of piperonylic acid (50 mg, 0.301 mmol, 1.0 eq.), [(pyridin-2-yl)methyl]-1H-pyrazol-4-amine (ZHAWOC8592) (52 mg, 0.301 mmol, 1 eq.) and *N*,*N*-diisopropylethylamine (DIPEA) (105 µl, 0.602 mmol, 2.0 eq.) in *N*,*N*-dimethylformamide (DMF) (2.5 ml) and the reaction mixture was stirred for 1 h. The reaction mixture was diluted with ethyl acetate (50 ml) and the organic layer washed with 1 N HCl (2 ×50ml), 1N NaOH (2 ×50ml) and saturated NaCl (2 × 50 ml). The organic layer was then dried with Na₂SO₄ and the solvent was removed under reduced pressure. The crude product was purified by silica gel flash chromatography using a gradient from dichloromethane to 20 % methanol in dichloromethane to afford *N*-{1-[(pyridin-2-yl)methyl]-1H-pyrazol-4-yl}-2H-1,3-benzodioxole-5-carboxamide (55 mg, 0.171 mmol, 57 %).
¹H NMR (500 MHz, DMSO-*d₆*) δ 10.34 (s, 1H), 8.54 (ddd, *J* = 4.8, 1.8, 0.9 Hz, 1H), 8.23 - 8.13 (m, 1H), 7.77 (ddd, *J* = 7.7, 7.7, 1.8 Hz, 1H), 7.66-7.62 (m, 1H), 7.55 (dd, *J* = 8.2, 1.8 Hz, 1H), 7.50-7.47 (m, 1H), 7.32 (ddd, *J* = 7.5, 4.8, 1.1 Hz, 1H), 7.07-7.04 (m, 1H), 7.04 - 7.01 (m, 1H), 6.13 (s, 2H), 5.42 (s, 2H).
¹³C NMR (126 MHz, DMSO-*d₆*) δ 163.1, 157.4, 150.4, 149.6, 147.9, 137.6, 131.4, 128.5, 123.2, 122.9, 122.7, 122.1, 122.0, 108.5, 107.8, 102.3, 57.2.

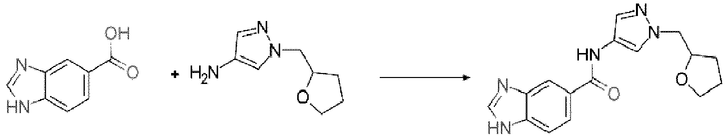

### N-{1-[(oxolan-2-yl)methyl]-1H-pyrazol-4-yl}-1H-1,3-benzodiazole-5-carboxamide, ZHAWOC8597 (Compound 10)

5-benzimidazolecarboxylic acid (100 mg, 0.617 mmol, 1.0 eq.) and 4-amino-1-[(oxolan-2-yl)methyl]-1H-pyrazole (ZHAWOC8590) (103 mg, 0.617 mmol, 1.0 eq.) were treated similar to the procedure of *N*-{1-[(pyridin-2-yl)methyl]-1H-pyrazol-4-yl}-2H-1,3-benzodioxole-5-carboxamide (ZHAWOC8595) to yield *N*-{1-[(oxolan-2-yl)methyl]-1H-pyrazol-4-yl}-1H-1,3-benzodiazole-5-carboxamide (82 mg, 0.263 mmol, 43 %).
¹H NMR (500 MHz, MeOD-*d₄*) δ 8.35 - 8.33 (m, 1H), 8.27 (s, 1H), 8.14 (d, *J* = 0.6 Hz, 1H), 7.90 (dd, *J* = 8.5, 1.6 Hz, 1H), 7.76 - 7.67 (m, 2H), 4.30 - 4.23 (m, 2H), 4.18 (dd, *J* = 14.9, 7.3 Hz, 1H), 3.95 (s, 1H), 3.92 - 3.86 (m, 1H), 3.80 - 3.74 (m, 1H), 2.09 - 2.00 (m, 1H), 1.94 - 1.77 (m, 2H), 1.73 - 1.64 (m, 1H). (One protic hydrogen undergoes a deuterium exchange.)
¹³C NMR (126 MHz, DMSO-*d₆*) δ 167.3, 164.4, 144.8, 144.2, 143.1, 130.7, 122.5, 121.8, 121.3, 119.0, 111.9, 77.8, 67.8, 55.8, 28.7, 25.5.

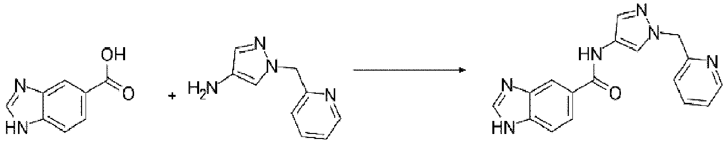

### N-{1-[(pyridin-2-yl)methyl]-1H-pyrazol-4-yl}-1H-1,3-benzodiazole-5-carboxamide, ZHAWOC8605 (Compound 11)

5-benzimidazolecarboxylic acid (100 mg, 0.617 mmol, 1.0 eq.) and [(pyridin-2-yl)methyl]-1H-pyrazol-4-amine (ZHAWOC8592) (234 mg, 0.617 mmol, 1.0 eq.) were treated similar to the procedure of *N*-{1-[(pyridin-2-yl)methyl]-1H-pyrazol-4-yl}-2H-1,3-benzodioxole-5-carboxamide (ZHAWOC8595) to yield *N*-{1-[(pyridin-2-yl)methyl]-1H-pyrazol-4-yl}-1H-1,3-benzodiazole-5-carboxamide (11 mg, 0.0346 mmol, 6 %).
¹H NMR (500 MHz, DMSO-*d₆*) δ 12.84 (s, 1H), 10.48 (s, 1H), 8.56 (ddd, *J=* 4.8, 1.8, 0.9 Hz, 1H), 8.38 (s, 1H), 8.28 (s, 1H), 8.22 (d, *J* = 0.6 Hz, 1H), 7.86 (dd, *J* = 8.5, 1.7 Hz, 1H), 7.78 (ddd, *J* = 7.7, 7.7, 1.8 Hz, 1H), 7.70-7.67 (m, 2H), 7.32 (ddd, *J* = 7.5, 4.8, 1.1 Hz, 1H), 7.05 (ddd, *J* = 7.9, 1.0, 1.0 Hz, 1H), 5.44 (s, 2H).
¹³C NMR (126 MHz, DMSO-*d₆*) δ 164.5, 157.5, 151.6, 149.6, 144.5, 137.6, 131.5, 129.3, 128.4, 123.2, 122.9, 122.1, 122.1, 121.2, 115.2, 115.9, 57.3.

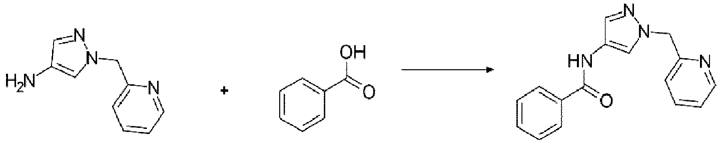

### N-{1-[(pyridin-2-yl)methyl]-1H-pyrazol-4-yl}benzamide, ZHAWOC8601 (Compound 12)

Benzoic acid (100 mg, 0.819 mmol, 1.0 eq.) and 4-amino-1-[(oxolan-2-yl)methyl]-1H-pyrazole (ZHAWOC8590) (143 mg, 0.819 mmol, 1.0 eq.) were treated similar to the procedure of *N*-{1-[(pyridin-2-yl)methyl]-1H-pyrazol-4-yl}-2H-1,3-benzodioxole-5-carboxamide (ZHAWOC8595) to yield *N*-{1-[(pyridin-2-yl)methyl]-1H-pyrazol-4-yl}benzamide (105 mg, 0.351 mmol, 59 %).
¹H NMR (500 MHz, MeOD-*d₄*) δ 8.63 - 8.55 (m, 1H), 8.25 (s, 1H), 7.97 - 7.88 (m, 3H), 7.75 (s, 1H), 7.59 - 7.55 (m, 1H), 7.53 - 7.44 (m, 3H), 7.26 - 7.20 (m, 1H), 5.52 (s, 2H). (the protic hydrogen undergoes a deuterium exchange.)
¹³C NMR (126 MHz, MeOD-*d₄*) δ 165.9, 155.5, 147.5, 139.5, 133.9, 131.8, 131.6, 129.3, 128.3, 128.1, 127.1, 123.6, 122.7, 122.5, 122.4, 55.7.

### Z20 Z990889372 (Enamine Library compound)

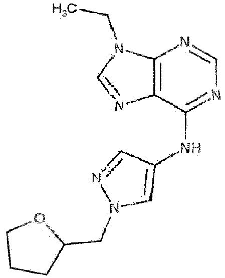

### Z28 Z729060316 (Enamine Library compound)

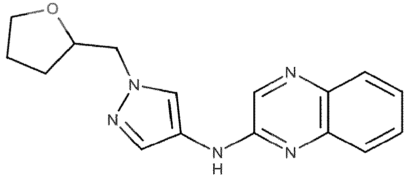

### Z18 Z2093528556 (Enamine Library compound)

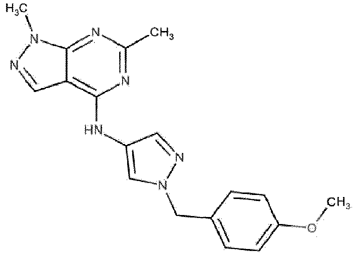

### RESULTS

### Cse expression regulates heterotopic calcification and cartilage damage in murine knee joints

To investigate the role *of Cse* in the pathogenesis of knee HC, we first used the surgical mouse model of knee medial meniscectomy (MNX). This model is characterized not only by knee HC but also by cartilage damage recapitulating osteoarthritis. Firstly, we observed that *Cse* expression was higher in sham-operated healthy knees than in osteoarthritic MNX knees (Figure **1a**). Given this observation, we decided to compare HC and cartilage damage in WT vs mice with altered *Cse* expression (either *Cse* KO or *Cse* tg mice). By CT-scan analysis we observed that, at 2 months post-surgery, operated knees from *Cse*-deficient mice *(Cse* KO) had increased peri- and intra-articular HC compared to WT littermates (Figure **1a**, white arrows). Quantitative analysis revealed that the volume of the HC, as well as their overall crystal content, was markedly increased in knee joints from *Cse* KO mice (Figure **1b**).

Next, the effect of *Cse* deficiency on cartilage damage and proteoglycan content was assessed in menisectomized mice by Safranin-O staining and quantification by OARSI scores. Pictures in Figure **1c** show that cartilage damage (fissuring and fibrillation), as well as proteoglycan loss (reduced red Safranin-O staining) were exacerbated in *Cse* KO joints compared to the WT ones. Likewise, OARSI scores for cartilage damage and Safranin-O loss were significantly increased in cartilage from *Cse* KO mice (Figure **1d**). This increased cartilage damage could not be accounted for by increased chondrocyte apoptosis, as immunohistochemical staining and quantification of apoptotic chondrocytes in murine knee sections revealed equivalent numbers of apoptotic chondrocyte in the different genotypes. We confirm the protective role of *Cse* in knee HC in aged mice. In old *Cse*-deficient mice (61-64 weeks old) we found by microCT-scan increased joint HC if compared to WT littermates. Indeed, HC volume and HC crystal content were higher in *Cse* KO, although these quantitative measurements did not reach significance. In addition, cartilage damage and Safranin-O loss were significantly increased in cartilage from *Cse* KO aged mice.

We then investigated the same readouts in mice overexpressing *Cse* (*Cse* tg). Contrarly to *Cse* KO mice, they showed less severe surgically-induced HC as shown by CT-scan 3D reconstructions (Figure **1e**) and quantitative analysis (Figure **1f**). Concomitantly, cartilage damage was significantly reduced, as shown by histological staining and scoring (Figure **1g**, **1h**). Moreover, *Cse* overexpression did not alter significantly the number of apoptotic chondrocytes.

Altogether, these results demonstrate that *Cse* is a crucial enzyme involved in joint protection against HC and cartilage damage.

To assess how *Cse* might interfere with cartilage degradation, we measured the expression of genes encoding for matrix-degrading enzymes (*Mmp-3* and *Mmp-13*) and for inhibitors of matrix-degrading enzymes (*Timp-1* and *Timp-3*) in WT, *Cse* KO and *Cse* tg chondrocytes at basal level. *Cse* KO cells showed significantly increased expression of *Mmp-3* and *Mmp-13,* and decreased *Timp-1* expression relative to WT cells (Figure **1i**). On the contrary, *Mmp-3* and *Mmp-13* expression levels were decreased and *Timp-3* strongly increased in *Cse* tg chondrocytes. The fact that *Cse* KO cells expressed more matrix-degrading enzymes and less corresponding inhibitors is in line with increased cartilage degradation observed *in vivo* in the MNX model of HC (Figure **1a****-d**). Similarly, less severe phenotypes seen in *Cse* tg mice subjected to the same model (Figure **1e****-h**), could be due to lower basal expression of matrix proteases and increased metalloproteases inhibitors.

Overall, we concluded that the pro-catabolic related gene expression signature was attenuated by endogenous *Cse*-generated H₂S.

### Endogenously Cse-generated H₂S regulates chondrocyte calcification and IL-6 secretion in vitro

To further test the role of F₂S in preventing chondrocyte calcification, we assessed endogenous H₂S levels in chondrocytes cultured in calcification medium for up to 10 days using the H₂S-specific fluorescent probe P3 by FACS (described in (Singha et al. 2015)) . While chondrocyte calcification started to appear massively at 7 days of culture and increased over-time (Figure **2a**), H₂S levels were reduced by approximately 40% after 7 days and by 60% after 10 days (Figure **2b**). In line with these findings, chondrocytes stimulated with HA crystals showed down-regulation *of Cse* expression (Figure **2c**).

We next explored the relationship between chondrocyte calcification and expression of H₂S-producing enzymes, using cells that were *Cse* deficient (*Cse* KO) or *Cse* overexpression (*Cse* tg). WT chondrocytes expressed Cse mRNA (Ct value of 28), together with the other two H2S-producing enzymes *3-Mst* and *Cbs* (Ct values of 28 for *Cbs,* 26 for *3-Mst*)*.* In *Cse-*deficient chondrocytes, *Cse* expression was undetectable, and associated with a decrease of 3-*Mst* expression, whereas *Cbs* expression was similar to that of WT chondrocytes (Figure **2d**). Conversely, chondrocytes from *Cse* tg mice showed the expected increase in *Cse* expression associated with a 7-fold increase in *3-Mst* expression, whereas *Cbs* expression was similar relative to WT chondrocytes (Figure **2d**). Taken together, these results suggest that expression of *Cse* and *3-Mst* are coordinated, without any evidence for compensation by *Cbs* in the absence of *Cse.*

We then demonstrated that *Cse* KO chondrocytes produced significantly less H₂S, while *Cse* tg chondrocytes produced significantly more H₂S, when compared to WT control chondrocytes (Figure **2e**).

To confirm the role of endogenously *Cse*-produced H₂S in inhibiting chondrocyte calcification, we isolated chondrocytes from WT, *Cse* KO and *Cse* tg mice, and cultured them for 24 hours in calcifying medium (CPP). Calcification was massively increased in *Cse* KO chondrocytes compared to WT cells, as demonstrated by Alizarin red staining of calcium-containing crystals and by quantification of calcium content in cell monolayer (Figure **2f**). As we observed previously that IL-6 was a pro-calcification cytokine produced by chondrocytes (Nasi, So, et al. 2016), we measured IL-6 in chondrocytes supernatant. We found that the massive pro-calcification phenotype of *Cse* KO chondrocytes was associated with significantly increased levels of basal IL-6 secretion (Figure **2g**). Conversely, *Cse* tg chondrocytes showed significantly decreased mineralization (Figure **2h**) and IL-6 secretion (Figure **2i**) compared to WT.

We then examined the effect of H₂S on the calcification enzyme *Alpl.* Basal *Alpl* activity in *Cse* KO chondrocytes was significantly increased relative to WT cells, while *Cse* tg cells showed no difference (Figure **2l**), suggesting that the hyper-calcification phenotype seen in *Cse*-deficient chondrocytes could be at least partly, due to increased *Alpl* activity.

*Cse* loss also affected chondrocyte differentiation (hypertrophy) associated with calcification. *Cse* KO chondrocytes preferentially expressed hypertrophic related genes such as *Coll10* and *Runx2,* as determined by qRT-PCR (ratio *Coll10*/*Coll2:* 3 in *Cse* KO cells, 1 in WT cells; ratio *Runx2*/*Sox9:* 1 in *Cse* KO cells, 0.6 in WT cells).

Increased levels of homocysteine have been reported in *Cse*-deficient mice and cells (Ishii et al. 2010). As elevated chondrocyte homocysteine levels may induce mitochondrial dysfunction, apoptosis, and oxidative stress (Ma et al. 2018), we wanted to rule out that the effects described above in *Cse*-deficient cells could be accounted for by increased homocysteine levels. When WT chondrocytes were incubated with two different concentrations of homocysteine (100µM or 25µM), calcification was significantly decreased (Figure **2m**), therefore excluding a role of hyperhomocysteinemia in the exacerbated calcification phenotype of *Cse*-deficient chondrocytes.

### Cse expression regulates Achilles tendon heterotopic calcification

To investigate the role *of Cse* in the pathogenesis of tendon HC, we first used the mouse model of Achilles tendon tenotomy. By CT-scan analysis we observed that, at 6 weeks post-surgery, operated tendons showed massive HC that increased over-time (Figure 3a, white arrows). Quantitative analysis confirmed increased HC volume from 4 weeks to 16 weeks post-surgery (Figure 3b).

Very interestingly, we found a striking reduction of CSE expression in the corresponding dissected tendons over time. In particular, at 1 week post-surgery the pink CSE staining was homogenously distributed in tendon cells, at 4 weeks it started to localize in specific points within chondrocyte-like cells, until complete disappearance at 16 weeks post-surgery (Figure **3c, 3d**). We confirmed the protective role *of Cse* in Achilles tendon HC in aged mice. In old *Cse*-deficient mice (35 weeks old) we found by microCT-scan increased Achilles tendon HC if compared to WT littermates (Figure **3e**, white arrows). Indeed, HC volume, HC crystal content and HC trabecular thickness Tb.Th were significantly higher in *Cse* KO (Figure **3f**). Finally, we isolated primary murine tenocytes from WT and *Cse* KO mice and tested their calcification level upon 24 hours in culture with calcifying particles. We found exacerbated calcium-containing crystals deposition in *Cse* KO cultures compared to WT cultures, as assessed by Alizarin Red staining and quantification (Figure **3g**). Additionally, the expression of pro-calcification and osteogenic genes such as bone sialoprotein (*Bsp*)*,* alkaline phosphatase (*Alpl*), and bone morphogenetic proteins-2 and -4 (*Bmp-2, Bmp-4*), known to be upregulated in calcific tendinopathy, was significantly augmented in *Cse* KO tenocytes if compared to WT (Figure **3h**).

Altogether, these results demonstrated that the negative correlation between HC and *Cse* expression is a general feature that does not only exist in knee cartilage (Figure **1**) but also in tendons.

### CSE expression in human cartilage and tendons negatively correlates with tissue calcification and disease severity.

We then wanted to expand our findings to human samples. In a series of 14 OA patients undergoing knee joint replacement (K/L grade 3-4), *CSE* expression assessed by qPCR was found in all cartilage specimens and was decreased if compared to healthy cartilage, even though this did not reach statistical significance (p=0.1, data not shown). By immunohistochemistry (Figure **4a**), *CSE* was found to be expressed mainly by chondrocytes in the superficial and tangential layer of cartilage while little expression was seen in deep cartilage and close to the tidemark with the bone. Contrarily, von Kossa staining for chondrocyte calcification was mostly positive in deep cartilage areas with rare extracellular crystal deposits in the upper zones, thus indicating that in OA cartilage *CSE* expression and calcification are mutually exclusive. Interestingly, we found a significant (p=0.03) inverse (r=-0.57) correlation between CSE expression and chondrocyte calcification (graph Figure **4a**). To confirm this observation, cartilage specimens from OA patients were subdivided into low, medium or high OA, according to radiographic (K/L from 1 to 4) and OARSI (from 0 to 5) scores (Low OA group: K/L 1-2 and OARSI 2-3, age 70.25 ± 5.37 years, medium OA group: K/L 3 and OARSI 3-4, 65.25 ± 14.88 years and high OA group: K/L 4 and OARSI 5, 59.8 ± 9,33 years). While *CSE* expression was decreased by 30% in medium and high OA (Figure **4b**, upper panel), we found that the extent of cartilage calcification, assessed by von Kossa staining, increased in more severe OA (Figure **4b**, lower panel). To further gain mechanistic insights into the negative correlation between *CSE* levels and the extent of cartilage calcification in OA, we incubated macroscopically non-eroded cartilage explants from 4 patients with HA crystals for 24h (Figure **4c**). *CSE* expression, as assessed by immunohistochemical staining and quantification, significantly decreased following incubation with crystals (graph Figure **4c**). Altogether, these results indicate that cartilage calcification increased during OA progression and the presence of HA crystals further decreased *CSE* expression in proportion of disease severity.

Finally, we analyzed CSE expression in a series of 19 patients with calcific tendinopathy (TC) undergoing excision of a portion of calcified tendon. Patients were classified into low, medium or high TC according to calcification score (Low TC group: calcification <1% isolated tissue, age 51.14 ± 14.84 years; medium TC group: calcification 1% to 20% isolated tissue, 58.43 ± 12.22 years and high TC group: calcification > 20% isolated tissue, 54 ± 7.65 years). As in murine tendons (Figure **3a****, c**), *CSE* expression was found to be inversely correlated to the degree of tendon calcification. Indeed, *CSE* was highly expressed in tendons from patients with low TC, while it significantly decreased in high TC patients (Figure **4d**).

### A new CSE activator compound prevents chondrocyte calcification and IL-6 secretion in murine and human chondrocytes

All the above results demonstrated that down-modulation of *CSE* in chondrocyte or tenocytes, favored cell calcification, IL-6 secretion, and cartilage matrix catabolism (Figures **1-4**). We therefore hypothesized that potential *CSE* activators could counter these effects and be of therapeutic value in musculoskeletal HC. We set up a screening assay with human recombinant *CSE* (*hCSE*) and L-Cysteine as substrate, using as read-out the production of pyruvate as a surrogate of H₂S production. As expected, pyruvate was detected only in presence of hCSE. Additionally, pyruvate up-modulation was observed in the presence of *CSE* and of an already described activator of the enzyme, calmodulin (Yang et al. 2008), whereas an almost complete inhibition was observed in the presence of AVG, a known CSE inhibitor (Szabo and Papapetropoulos 2017). Based on these results, we screened 75000 compounds from a lead-like small molecule library. We identified one compound, named SU004, which increased pyruvate in a dose-dependent way with an estimated EC50 of 17µM. This compound was also efficient in a secondary screen based on H₂S detection by the P3 probe, with a similar EC50 (results not shown).

In cell assays using murine primary chondrocytes, this compound potently inhibited mineralization (Figure **5a**), with no cell toxicity, as demonstrated by low or undetectable LDH release (Figure **5b**). Interestingly, the inhibitory capacity of SU004 was similar to that of STS, which was used as a positive control (Figure **5a**). The specificity of this compound for *CSE* was confirmed using *Cse*-deficient chondrocytes, as *Cse*-deficient cells showed no decreased calcification while the compound exerted a strong inhibitory effect on calcification of WT chondrocyte (Figure **5a**). Finally, SU004 also inhibited IL-6 secretion at the same extent of the positive control STS (Figure **5c**). We then confirmed the inhibitory effects of SU004 also on human primary chondrocytes from OA patients. In cell culture from three independent patients, we found decreased calcification (Figure **5d**) and decreased IL-6 secretion (Figure **5f**) with no effect on cell cytotoxicity (Figure **5e**).

### Compound plate preparation

A set of 75 000 compounds from a lead-like small molecule library (Exquiron library, Reinach, Switzerland) was used. For screening purpose, the compound stock solutions were transferred from Labcyte 384 LDVplates (LP-0200) to 384 polypropylene plates (Greiner # 781201) using an Echo 555 acoustic liquid handler (LabCyte). The compound solutions (50 nl) were dispensed into columns 1-22 and an equivalent volume of DMSO, and a CSE inhibitor (AVG at 5 µM) used in columns 23 and 24, respectively. Plated compounds were stored at -20°C. The final compound concentration used in the screen was 50 µM, with a final DMSO concentration of 1% in all wells.

### High-throughput screening assay for CSE activators

For the robotic screening, a pyruvate assay was miniaturized and optimized on 384-well black polystyrene, flat bottom plates (Greiner). A mix containing 50nM recombinant human *CSE* (*hCSE,* ProteoGenix, France), 200µM L-Cysteine substrate (Sigma) in PBS + 1mM CaCl₂ with 0.1% BSA (Sigma) (Figure 7) was dispensed immediately to the assay plates containing compounds (at 50 µM in 1% DMSO final). After 3h at 37°C, pyruvate detection was performed as described, adding 0.25 U/ml pyruvate oxidase (POX, Sorachim), 0.2U/mL HRP (Sigma P8375), 25µM Ampliflu Red (Sigma 90101) in 100mM potassium phosphate pH 6.7 containing 1mM EDTA, 1mM MgCl₂, 10 µM FAD (Sigma F6625), 0.2mM TPP (Sigma C8754). 3 volumes of mix for pyruvate detection were used for 1 volume of mix with *hCSE.* The plates were incubated for one hour at ambient temperature. The resulting fluorescence signal was measured on En Vision (PerkinElmer) multiplate reader with Excitation 531nm Emission 595nm, with cutoff at 555nm. As a positive control we used *hCSE* at 500nM final concentration to assess the assay's quality and plate-to-plate reproducibility. The assay quality was assessed by Z' factor, which was > 0.5 using the following formula: Z'-factor=1-[(3(σp+σn)/(|µp-µn|)], where µn and σn represent the mean and standard deviation of the negative control (50nM *CSE*), and µp and σp represent the mean and standard deviation of the positive control (500nM *CSE*). Data were normalized to 0% activation (50nM *CSE*) and 100% activation (500nM *CSE*). Activators above 10% of normalized activity were selected.

### CBS is lowly expressed in human and murine cartilage and plays a minor role in chondrocyte calcification

We firstly assessed *CBS* expression by immunohistochemistry in both human and murine knee cartilage. In human cartilage, few positive cells for *CBS* expression were present in superficial cartilage layer, while intermediate and deep layers remained negative. In murine healthy cartilage, superficial chondrocytes expressed *CBS.* Following induction of experimental HC and OA by menisectomy, *CBS* expression was strongly decreased. *Cbs-*deficient chondrocytes (*Cbs* KO) exhibited the same characteristics of *Cse* KO cells (i.e increased calcification and increased IL-6 production) although the amounts of the formed crystals and of secreted IL-6 were much smaller (9-fold less and 12-fold less respectively compared to the results obtained in *Cse* KO cells) (Figure 2e, 2f). Interestingly, *Cbs* KO chondrocytes showed concomitant increase of both *Cse* and *3-Mst* expression if compared to WT chondrocytes. Finally, H₂S levels in *Cbs* KO chondrocytes were similar to those of the corresponding WT chondrocytes (Figure 5f) potentially due to compensated production of the other two H₂S-producing enzymes.

### C5, C6 and Z20 results discussion

We assessed these 3 compounds for their activities on H₂S production and on calcification inhibition in different cell types. All three demonstrated anti-calcification properties in a dose dependent manner (2-50uM) in chondrocytes (human and murine primary cells as well as cell lines), vascular smooth muscle (MOVAS cell line). In addition, Z20 inhibited calcification in tenocytes (primary) and keratinocytes (HaCaT cell line). Specificity for CSE was demonstrated when Z20 was added to primary vascular smooth muscle cells derived from wild type or CSE *ko* mice.

A significant inhibition was seen in wild type cells, whereas in CSE *ko* cells, calcification was augmented and no inhibition was seen when Z20 was added.

### REFERENCES

Aghagolzadeh, P., R. Radpour, M. Bachtler, H. van Goor, E. R. Smith, A. Lister, A. Odermatt, M. Feelisch, and A. Pasch. 2017. 'Hydrogen sulfide attenuates calcification of vascular smooth muscle cells via KEAP1/NRF2/NQ01 activation', Atherosclerosis, 265: 78-86.
Akahoshi, N., C. Kobayashi, Y. Ishizaki, T. Izumi, T. Himi, M. Suematsu, and I. Ishii. 2008. 'Genetic background conversion ameliorates semi-lethality and permits behavioral analyses in cystathionine beta-synthase-deficient mice, an animal model for hyperhomocysteinemia', Hum Mol Genet, 17: 1994-2005.
Bai, G., D. S. Howell, G. A. Howard, B. A. Roos, and H. S. Cheung. 2001. 'Basic calcium phosphate crystals up-regulate metalloproteinases but down-regulate tissue inhibitor of metalloproteinase-1 and -2 in human fibroblasts', Osteoarthritis Cartilage, 9: 416-22.
Burguera, E. F., R. Meijide-Failde, and F. J. Blanco. 2017. 'Hydrogen Sulfide and Inflammatory Joint Diseases', Curr Drug Targets, 18: 1641-52.
Burguera, E. F., A. Vela-Anero, J. Magalhaes, R. Meijide-Failde, and F. J. Blanco. 2014. 'Effect of hydrogen sulfide sources on inflammation and catabolic markers on interleukin 1beta-stimulated human articular chondrocytes', Osteoarthritis Cartilage, 22: 1026-35.
Campillo-Gimenez, L., F. Renaudin, P. Bobé, M. Gosset, C. Combes, M. Cohen-Solal, F. Lioté, and H.-K. Ea. 2016. 'OP0287 Calcium Pyrophosphate Dihydrate Crystals Induce IL-1β Production by Monocytes through A Potassium Efflux-Dependent Pathway', Annals of the Rheumatic Diseases, 75: 166-67.
Campillo-Gimenez, L., F. Renaudin, M. Jalabert, P. Gras, M. Gosset, C. Rey, S. Sarda, C. Collet, M. Cohen-Solal, C. Combes, F. Liote, and H. K. Ea. 2018. 'Inflammatory Potential of Four Different Phases of Calcium Pyrophosphate Relies on NF-kappaB Activation and MAPK Pathways', Front Immunol, 9: 2248.
Desai, J., O. Foresto-Neto, M. Honarpisheh, S. Steiger, D. Nakazawa, B. Popper, E. M. Buhl, P. Boor, S. R. Mulay, and H. J. Anders. 2017. 'Particles of different sizes and shapes induce neutrophil necroptosis followed by the release of neutrophil extracellular trap-like chromatin', Sci Rep, 7: 15003.
Ea, H. K., V. Monceau, E. Camors, M. Cohen-Solal, D. Charlemagne, and F. Liote. 2008. 'Annexin 5 overexpression increased articular chondrocyte apoptosis induced by basic calcium phosphate crystals', Ann Rheum Dis, 67: 1617-25.
Easley, R. A., M. C. Patsavas, R. H. Byrne, X. Liu, R. A. Feely, and J. T. Mathis. 2013. 'Spectrophotometric measurement of calcium carbonate saturation states in seawater', Environ Sci Technol, 47: 1468-77.
Foreman, S. C., A. S. Gersing, C. E. von Schacky, G. B. Joseph, J. Neumann, N. E. Lane, C. E. McCulloch, M. C. Nevitt, and T. M. Link. 2020. 'Chondrocalcinosis is associated with increased knee joint degeneration over 4 years: data from the Osteoarthritis Initiative', Osteoarthritis Cartilage, 28: 201-07.
Fox, B., J. T. Schantz, R. Haigh, M. E. Wood, P. K. Moore, N. Viner, J. P. Spencer, P. G. Winyard, and M. Whiteman. 2012. 'Inducible hydrogen sulfide synthesis in chondrocytes and mesenchymal progenitor cells: is H2S a novel cytoprotective mediator in the inflamed joint?', J Cell Mol Med, 16: 896-910.
Fuerst, M., J. Bertrand, L. Lammers, R. Dreier, F. Echtermeyer, Y. Nitschke, F. Rutsch, F. K. Schafer, O. Niggemeyer, J. Steinhagen, C. H. Lohmann, T. Pap, and W. Ruther. 2009. 'Calcification of articular cartilage in human osteoarthritis', Arthritis Rheum, 60: 2694-703.
Gregory, C. A., W. G. Gunn, A. Peister, and D. J. Prockop. 2004. 'An Alizarin red-based assay of mineralization by adherent cells in culture: comparison with cetylpyridinium chloride extraction', Anal Biochem, 329: 77-84.
Hashimoto, S., R. L. Ochs, F. Rosen, J. Quach, G. McCabe, J. Solan, J. E. Seegmiller, R. Terkeltaub, and M. Lotz. 1998. 'Chondrocyte-derived apoptotic bodies and calcification of articular cartilage', Proc Natl Acad Sci U S A, 95: 3094-9.
Hughes, M. N., M. N. Centelles, and K. P. Moore. 2009. 'Making and working with hydrogen sulfide: The chemistry and generation of hydrogen sulfide in vitro and its measurement in vivo: a review', Free Radic Biol Med, 47: 1346-53.
Ishii, I., N. Akahoshi, H. Yamada, S. Nakano, T. Izumi, and M. Suematsu. 2010. 'Cystathionine gamma-Lyase-deficient mice require dietary cysteine to protect against acute lethal myopathy and oxidative injury', J Biol Chem, 285: 26358-68.
Kamekura, S., K. Hoshi, T. Shimoaka, U. Chung, H. Chikuda, T. Yamada, M. Uchida, N. Ogata, A. Seichi, K. Nakamura, and H. Kawaguchi. 2005. 'Osteoarthritis development in novel experimental mouse models induced by knee joint instability', Osteoarthritis Cartilage, 13: 632-41.
Kloesch, B., M. Liszt, G. Steiner, and J. Broll. 2012. 'Inhibitors of p38 and ERK1/2 MAPkinase and hydrogen sulphide block constitutive and IL-1beta-induced IL-6 and IL-8 expression in the human chondrocyte cell line C-28/12', Rheumatol Int, 32: 729-36.
Liu, Y. Z., A. P. Jackson, and S. D. Cosgrove. 2009. 'Contribution of calcium-containing crystals to cartilage degradation and synovial inflammation in osteoarthritis', Osteoarthritis Cartilage, 17: 1333-40.
Liu-Bryan, R., and F. Liote. 2005. 'Monosodium urate and calcium pyrophosphate dihydrate (CPPD) crystals, inflammation, and cellular signaling', Joint Bone Spine, 72: 295-302.
Ma, C. H., Y. C. Chiua, C. H. Wu, I. M. Jou, Y. K. Tu, C. H. Hung, P. L. Hsieh, and K. L. Tsai. 2018. 'Homocysteine causes dysfunction of chondrocytes and oxidative stress through repression of SIRT1/AMPK pathway: A possible link between hyperhomocysteinemia and osteoarthritis', Redox Biol, 15: 504-12.
McCarthy, G. M., and A. Dunne. 2018. 'Calcium crystal deposition diseases - beyond gout', Not Rev Rheumatol, 14: 592-602.
McCarthy, G. M., P. R. Westfall, I. Masuda, P. A. Christopherson, H. S. Cheung, and P. G. Mitchell. 2001. 'Basic calcium phosphate crystals activate human osteoarthritic synovial fibroblasts and induce matrix metalloproteinase-13 (collagenase-3) in adult porcine articular chondrocytes', Ann Rheum Dis, 60: 399-406.
Meyers, C., J. Lisiecki, S. Miller, A. Levin, L. Fayad, C. Ding, T. Sono, E. McCarthy, B. Levi, and A. W. James. 2019. 'Heterotopic Ossification: A Comprehensive Review', JBMR Plus, 3: e10172.
Michel, P. A., D. Kronenberg, G. Neu, J. Stolberg-Stolberg, A. Frank, T. Pap, M. Langer, M. Fehr, M. J. Raschke, and R. Stange. 2020. 'Microsurgical reconstruction affects the outcome in a translational mouse model for Achilles tendon healing', J Orthop Translat, 24: 1-11.
Morita, K., T. Miyamoto, N. Fujita, Y. Kubota, K. Ito, K. Takubo, K. Miyamoto, K. Ninomiya, T. Suzuki, R. Iwasaki, M. Yagi, H. Takaishi, Y. Toyama, and T. Suda. 2007. 'Reactive oxygen species induce chondrocyte hypertrophy in endochondral ossification', J Exp Med, 204: 1613-23.
Mulay, S. R., J. Desai, S. V. Kumar, J. N. Eberhard, D. Thomasova, S. Romoli, M. Grigorescu, O. P. Kulkarni, B. Popper, V. Vielhauer, G. Zuchtriegel, C. Reichel, J. H. Brasen, P. Romagnani, R. Bilyy, L. E. Munoz, M. Herrmann, H. Liapis, S. Krautwald, A. Linkermann, and H. J. Anders. 2016. 'Cytotoxicity of crystals involves RIPK3-MLKL-mediated necroptosis', Not Commun, 7: 10274.
Nasi, S., H. K. Ea, V. Chobaz, P. van Lent, F. Liote, A. So, and N. Busso. 2014. 'Dispensable role of myeloid differentiation primary response gene 88 (MyD88) and MyD88-dependent toll-like receptors (TLRs) in a murine model of osteoarthritis', Joint Bone Spine*.*
Nasi, S., H. K. Ea, F. Liote, A. So, and N. Busso. 2016. 'Sodium Thiosulfate Prevents Chondrocyte Mineralization and Reduces the Severity of Murine Osteoarthritis', PLoS One, 11: e0158196.
Nasi, S., D. Ehirchiou, A. Chatzianastasiou, N. Nagahara, A. Papapetropoulos, J. Bertrand, G. Cirino, A. So, and N. Busso. 2020. 'The protective role of the 3-mercaptopyruvate sulfurtransferase (3-MST)-hydrogen sulfide (H2S) pathway against experimental osteoarthritis', Arthritis Res Ther, 22: 49.
Nasi, S., A. So, C. Combes, M. Daudon, and N. Busso. 2015. 'Interleukin-6 and chondrocyte mineralisation act in tandem to promote experimental osteoarthritis', Ann Rheum Dis.
---. 2016. 'Interleukin-6 and chondrocyte mineralisation act in tandem to promote experimental osteoarthritis', Ann Rheum Dis, 75: 1372-9.
O'Brien, E. J., C. B. Frank, N. G. Shrive, B. Hallgrimsson, and D. A. Hart. 2012. 'Heterotopic mineralization (ossification or calcification) in tendinopathy or following surgical tendon trauma', Int J Exp Pathol, 93: 319-31.
Oliva, F., A. G. Via, and N. Maffulli. 2012. 'Physiopathology of intratendinous calcific deposition', BMC Med, 10: 95.
Olson, K. R. 2018. 'H2S and polysulfide metabolism: Conventional and unconventional pathways', Biochem Pharmacol, 149: 77-90.
Polhemus, D. J., and D. J. Lefer. 2014. 'Emergence of hydrogen sulfide as an endogenous gaseous signaling molecule in cardiovascular disease', Circ Res, 114: 730-7.
Pritzker, K. P., S. Gay, S. A. Jimenez, K. Ostergaard, J. P. Pelletier, P. A. Revell, D. Salter, and W. B. van den Berg. 2006. 'Osteoarthritis cartilage histopathology: grading and staging', Osteoarthritis Cartilage, 14: 13-29.
Prudhommeaux, F., C. Schiltz, F. Liote, A. Hina, R. Champy, B. Bucki, E. Ortiz-Bravo, A. Meunier, C. Rey, and T. Bardin. 1996. 'Variation in the inflammatory properties of basic calcium phosphate crystals according to crystal type', Arthritis Rheum, 39: 1319-26.
Ranganathan, K., S. Loder, S. Agarwal, V. W. Wong, J. Forsberg, T. A. Davis, S. Wang, A. W. James, and B. Levi. 2015. 'Heterotopic Ossification: Basic-Science Principles and Clinical Correlates', J Bone Joint Surg Am, 97: 1101-11.
Rose, P., P. K. Moore, and Y. Z. Zhu. 2017. 'H2S biosynthesis and catabolism: new insights from molecular studies', Cell Mol Life Sci, 74: 1391-412.
Ryu, J. H., S. Yang, Y. Shin, J. Rhee, C. H. Chun, and J. S. Chun. 2011. 'Interleukin-6 plays an essential role in hypoxia-inducible factor 2alpha-induced experimental osteoarthritic cartilage destruction in mice', Arthritis Rheum, 63: 2732-43.
Sikura, K. E., L. Potor, T. Szerafin, M. Oros, P. Nagy, G. Mehes, Z. Hendrik, A. Zarjou, A. Agarwal, N. Posta, R. Torregrossa, M. Whiteman, I. Furtos, G. Balla, and J. Balla. 2020. 'Hydrogen sulfide inhibits calcification of heart valves; implications for calcific aortic valve disease', Br J Pharmacol, 177: 793-809.
Singha, S., D. Kim, H. Moon, T. Wang, K. H. Kim, Y. H. Shin, J. Jung, E. Seo, S. J. Lee, and K. H. Ahn. 2015. 'Toward a selective, sensitive, fast-responsive, and biocompatible two-photon probe for hydrogen sulfide in live cells', Anal Chem, 87: 1188-95.
Stack, J., and G. M. McCarthy. 2020. 'Cartilage calcification and osteoarthritis: a pathological association?', Osteoarthritis Cartilage*.*
Szabo, C., and A. Papapetropoulos. 2017. 'International Union of Basic and Clinical Pharmacology. CII: Pharmacological Modulation of H2S Levels: H2S Donors and H2S Biosynthesis Inhibitors', Pharmacol Rev, 69: 497-564.
Vela-Anero, A., T. Hermida-Gomez, L. Gato-Calvo, C. Vaamonde-Garcia, S. Diaz-Prado, R. Meijide-Failde, F. J. Blanco, and E. F. Burguera. 2017. 'Long-term effects of hydrogen sulfide on the anabolic-catabolic balance of articular cartilage in vitro', Nitric Oxide, 70: 42-50.
Yan, J. F., W. P. Qin, B. C. Xiao, Q. Q. Wan, F. R. Tay, L. N. Niu, and K. Jiao. 2020. 'Pathological calcification in osteoarthritis: an outcome or a disease initiator?', Biol Rev Comb Philos Soc, 95: 960-85.
Yang, G., L. Wu, B. Jiang, W. Yang, J. Qi, K. Cao, Q. Meng, A. K. Mustafa, W. Mu, S. Zhang, S. H. Snyder, and R. Wang. 2008. 'H2S as a physiologic vasorelaxant: hypertension in mice with deletion of cystathionine gamma-lyase', Science, 322: 587-90.
Zavaczki, E., V. Jeney, A. Agarwal, A. Zarjou, M. Oros, M. Katko, Z. Varga, G. Balla, and J. Balla. 2011. 'Hydrogen sulfide inhibits the calcification and osteoblastic differentiation of vascular smooth muscle cells', Kidney Int, 80: 731-9.

## Claims

1. A compound of formula (I) 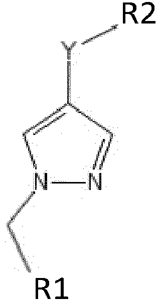 for use in the treatment and/or prevention of a condition associated with hypermineralization, wherein:
R1 is independently alkyl, aryl, cycloalkyl, heteroaryl, or heterocyclyl, optionally substituted;
R2 is nothing or independently cycloalkyl, heteroaryl, or heterocyclyl, optionally substituted; and
Y is NH, NH2, NO2, CH2 or 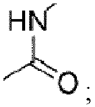
or a pharmaceutically acceptable salt, stereoisomer, mixture of stereoisomers, solvate, or tautomer thereof.

2. The compound of formula (I), wherein
R1 is independently 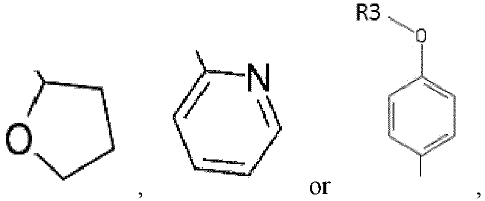 wherein R3 is an alkyl, preferably a C1-C6 alkyl; and
R2 is independently

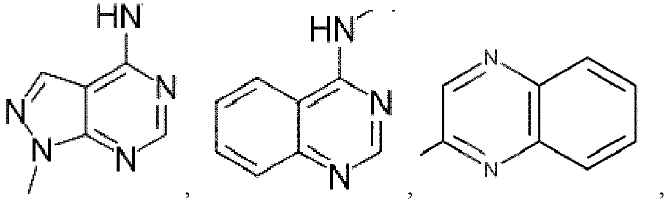

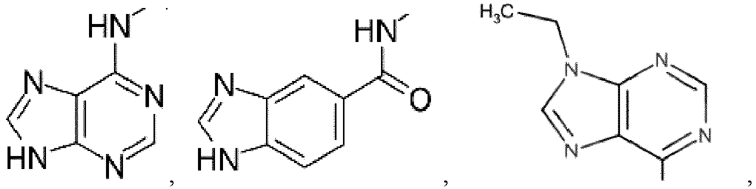

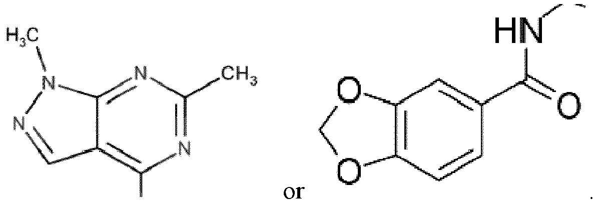

3. The compound of formula (I) according to any one of claims 1 to 2, selected from the the following compounds: 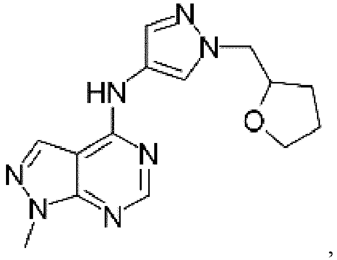 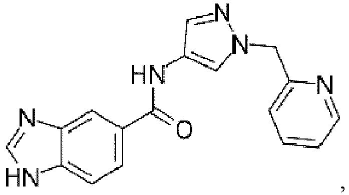 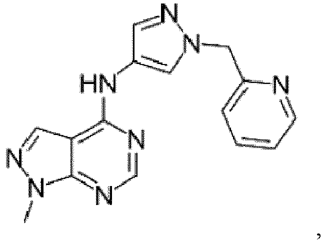 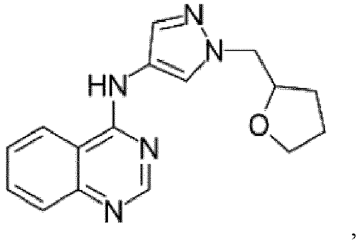 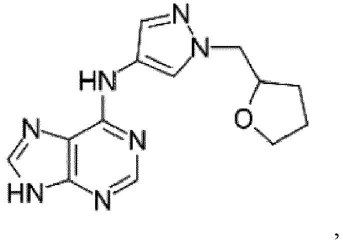 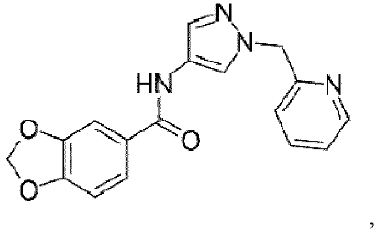 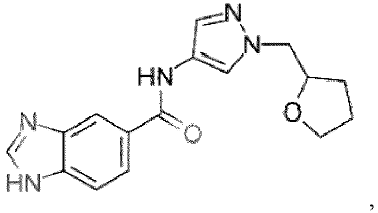 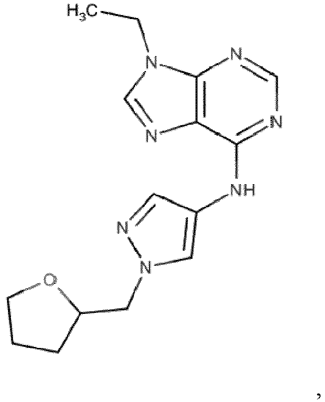 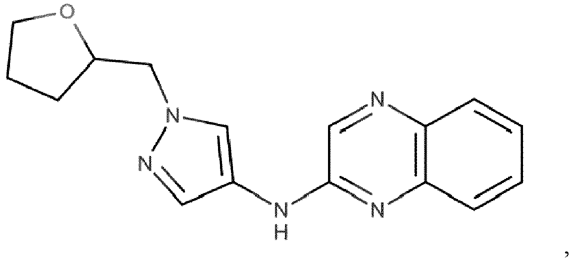 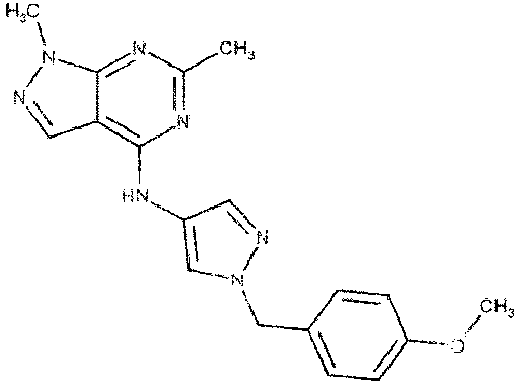 and

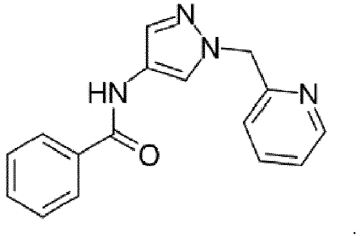

4. The compound of any one of claims 1 to 3, wherein the compound of formula (I) enhances the activity and/or expression of the cystathionine-gamma-lyase enzyme (CSE).

5. The compound of any one of claims 1 to 4, wherein the condition associated with hypermineralization isselected from the group comprising heterotopic calcification, osteoarthritis, medial vascular calcification, and ankylosis, or a combination of one or more of these conditions.

6. A pharmaceutical composition comprising a compound of anyone of claims 1-4, or a pharmaceutically acceptable salt thereof, together with a pharmaceutically acceptable carrier for use in the treatment and/or prevention of a condition associated with hypermineralization.

7. The pharmaceutical composition of claim 6, further comprising other therapeutic agents.

8. A compound of formula (I) 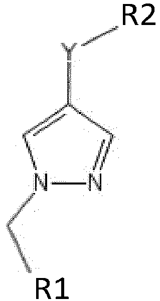 wherein:
R1 is independently alkyl, aryl, cycloalkyl, heteroaryl, or heterocyclyl, optionally substituted;
R2 is nothing or independently cycloalkyl, heteroaryl, or heterocyclyl, optionally substituted; and
Y is NH, NH2, NO2, CH2 or 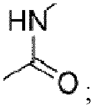
or a salt, stereoisomer, mixture of stereoisomers, solvate, or tautomer thereof.

9. The compound of formula (I) of claim 8, wherein
R1 is independently 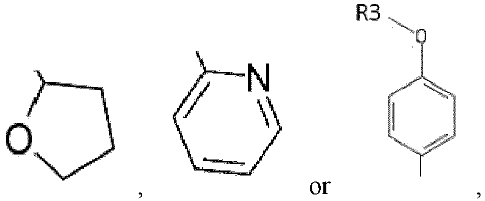 wherein R3 is an alkyl, preferably a C1-C6 alkyl; and
R2 is independently

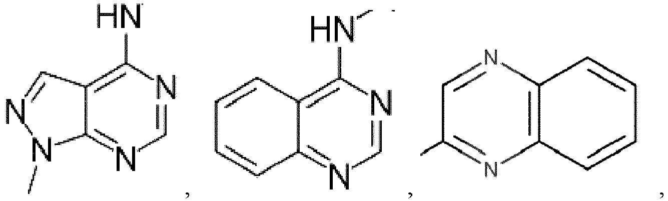

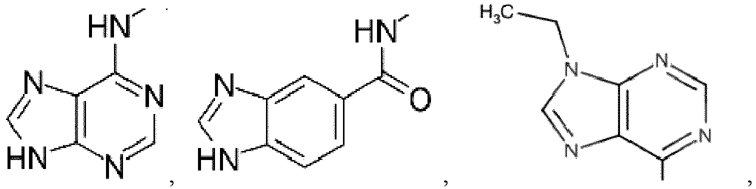

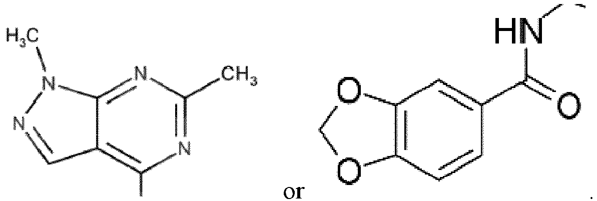

10. The compound of formula (I) according to any one of claims 8 to 9, selected from the following compounds: 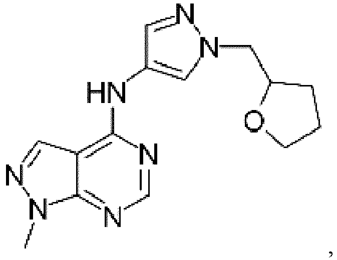 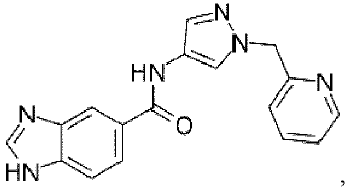 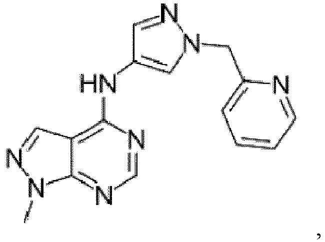 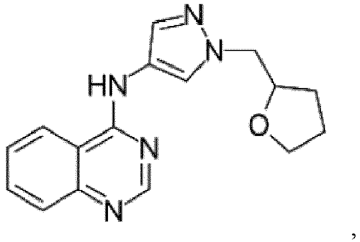 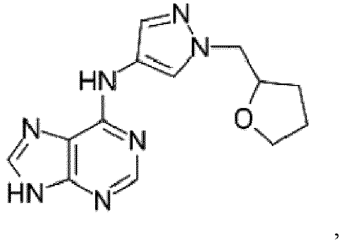 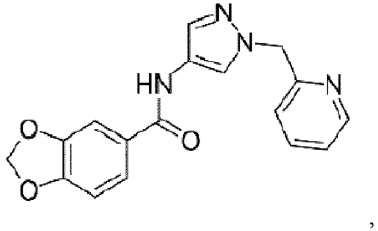 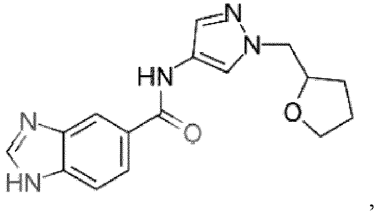 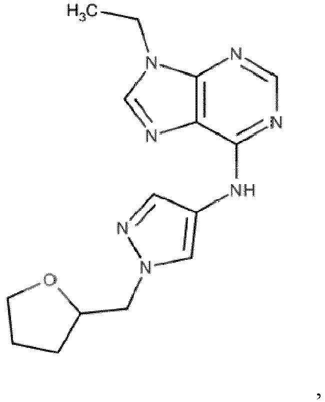 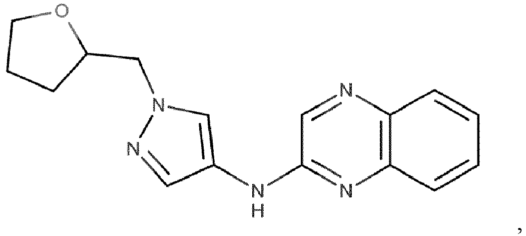 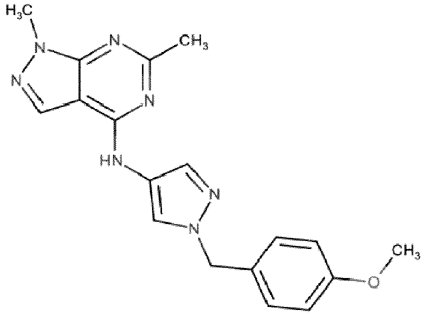 and
